# EUROPÄISCHE PATENTANMELDUNG

(11) **EP 1 939 182 A1**
(43) Veröffentlichungstag der Anmeldung: **02.07.2008**
(21) Anmeldenummer: 06127140.9
(22) Anmeldetag: 22.12.2006
(51) Int. Cl.: C07D 231/56, C07D 209/08, C07D 471/04

(54) **Verfahren zur Herstellung von (R oder S)-2-Alkyl-3-heterocyclyl-1-propanolen**

(71) Anmelder: Speedel Experimenta AG, 4123 Allschwil (CH)
(72) Erfinder: Die Erfindernennung liegt noch nicht vor
(74) Vertreter: Maué, Paul Georg

(57) **Zusammenfassung**

Verbindungen der Formel 1, 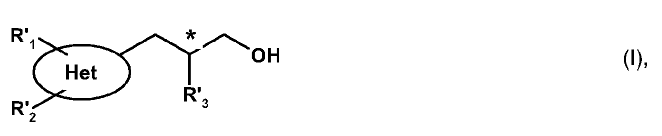 worin R'₁, R'₂,R'₃ und Het die Bedeutungen wie in der Beschreibung angegeben haben, sind in hohen Ausbeuten erhältlich durch eine stereoselektive Addition von R'₃-substiuierten Propionsäurestern an R'₁- und R'₂-substituierte ungesättigte, bicyclische Heterocyclylaldehyde der Formel R-CHO zu entsprechenden 3-R-3-Hydroxy-2-R'₃-propionsäureestern. Überführung der OH-Gruppe in eine Abgangsgruppe, eine anschliessend regioselektive Elimination zu 3-R-2-R'₃-propensäureestern, gefolgt von
1) der Verseifung zu den entsprechenden 3-R-2-R'₃-propensäuren, deren enantioselektive Hydrierung zu entsprechenden chiralen 3-R-2-R'₃-propensäuren und deren Reduktion, oder
2) der Verseifung zu den entsprechenden 3-R-2-R'₃-propensäuren, deren Reduktion zu entsprechenden 3-R-2-R'₃-Allylalkoholen und deren enantioselektive Hydrierung, oder
3) der Reduktion zu entsprechenden 3-R-2-R'₃-Allylalkoholen und deren enantioselektive Hydrierung, wobei R für 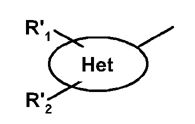 steht und die enantioselektiven Hydrierungen mit Metallkomplexen durchgeführt werden, welche als Liganden Ferrocen-1,1'-diphosphine, die in 1-Stellung eine Ferrocen-substituierte, sekundäre Phosphingruppe und in 1 '-Stellung eine sekundäre Phosphingruppe aufweisen, aufweisen.

## Beschreibung

Die Erfindung betrifft ein stereoselektives Verfahren zur Herstellung von (R oder S)-2-Alkyl-3-heterocyclyl-1-propanolen und neuer Zwischenprodukte, die bei den Verfahrensstufen erhalten werden.

In der WO 2005/090305 A1 werden δ-Amino-γ-hydroxy-ω-heterocyclyl-alkancarbonsäureamide beschrieben, die Renin-hemmende Eigenschaften aufweisen und als antihypertensive Mittel in pharmazeutischen Zubereitungen verwendet werden können. Die dort beschriebenen Herstellungsverfahren, die über eine Kupplung einer Heterocyclyl-Metall-Spezies an einen Aldehyd als Schlüsselschritt verlaufen, sind insbesondere hinsichtlich der zum Teil unbefriedigenden Ausbeuten nicht für ein industrielles Verfahren geeignet.

In einem neuen Verfahren geht man von 2,7-Dialkyl-8-heterocyclyl-4-octenoylamiden aus, deren Doppelbindung gleichzeitig in 5-Stellung halogeniert und in 4-Stellung unter Lactonisierung hydroxyliert wird, dann das Halogen mit Azid ersetzt, das Lacton amidiert und darauf das Azid in die Amingruppe überführt wird. Die gewünschten Alkancarbonsäureamide werden bei diesem neuen Verfahren in wesentlich höheren Gesamtausbeuten erhalten. Die Halolaktonisierung, die Azidierung und die Azidreduktion werden in Anlehnung an das von P. Herold im Journal of Organic Chemistry, Vol. 54 (1989), Seiten 1178-1185 beschriebene Verfahren durchgeführt.

Die 2,7-Dialkyl-8-heterocyclyl-4-octenoylamide können zum Beispiel der Formel A entsprechen, 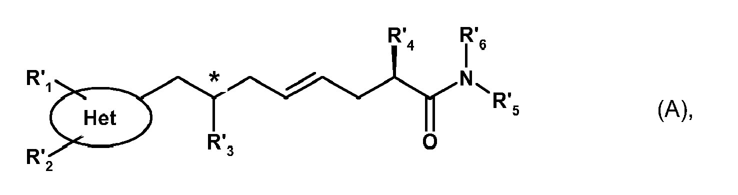 worin Het ein bicyclisches, über ein C-Atom an das Restmolekül geknüpftes, ungesättigtes Heterocyclyl bedeutet, wobei der nicht direkt an das Restmolekül gebundene Ring durch R'₁ und R'₂ substituiert ist, R'₁ und R'₂ unabhängig voneinander H, C₁-C₈-Alkyl, Halogen, Poly-halogen-C₁-C₈-alkoxy, Polyhalogen-C₁-C₈-alkyl, C₁-C₈-Alkoxy, C₁-C₈-Alkoxy-C₁-C₈-alkyl, oder C₁-C₈-Alkoxy-C₁-C₈-alkoxy darstellen, wobei R'₁ und R'₂ nicht gleichzeitig H bedeuten, R'₃ C₁-C₈-Alkyl bedeutet, R'₄ für C₁-C₈-Alkyl steht, R'₅ C₁-C₈-Alkyl oder C₁-C₈-Alkoxy bedeutet, R'₆ C₁-C₈-Alkyl darstellt, oder R'₅ und R'₆ zusammen gegebenenfalls mit C₁-C₄-Alkyl, Phenyl oder Benzyl substituiertes Tetramethylen, Pentamethylen, 3-Oxa-1,5-pentylen oder -CH₂CH₂O-C(O)- sind und worin das Kohlenstoffatom, an welches der Rest R'₃ gebunden ist, entweder (R)- oder (S)-Konfiguration aufweist, wobei die (R)-Konfiguration bevorzugt ist.

Die Verbindungen der Formel A sind erhältlich, indem man eine Verbindung der Formel B 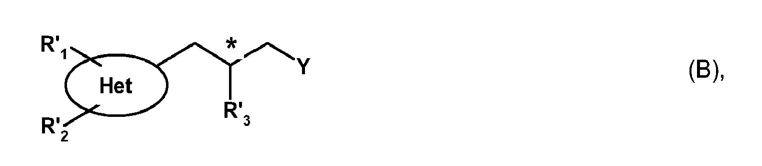 mit einer Verbindung der Formel C, 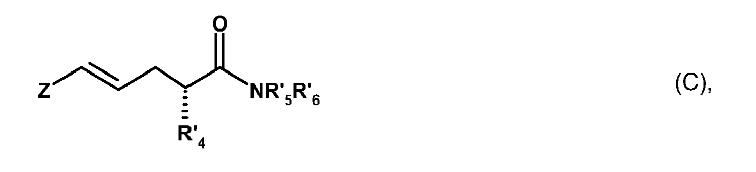 worin Het, R'₁ bis R'₄, R'₅ und R'₆ die zuvor angegebenen Bedeutungen haben, Y Cl, Br oder I und Z Cl, Br oder I bedeuten und worin das Kohlenstoffatom, an welches der Rest R'₃ gebunden ist, entweder (R)- oder (S)-Konfiguration aufweist, wobei die (R)-Konfiguration bevorzugt ist, in Gegenwart eines Alkali- oder Erdalkalimetalls umsetzt. Y und Z bedeuten bevorzugt Br oder Cl und besonders bevorzugt Cl.

Die Verbindungen der Formel C können durch Amidierung der entsprechenden Carbonsäureester, -amide oder -halogenide hergestellt werden. Die Bildung von Carbonsäureamiden aus Carbonsäureestern und Aminen in Gegenwart von Trialkylaluminium oder Dialkylaluminiumhalogenid, zum Beispiel mit Trimethylaluminium oder Dimethylaluminiumchlorid, ist von S. M. Weinreb in Organic Syntheses, 59, Seiten 49-53 (1980) beschrieben. Die Carbonsäureester sind erhältlich durch die Umsetzung von trans-1,3-Dihalogenpropen (zum Beispiel trans-1,3-Dichlorpropen) mit entsprechenden Carbonsäureestern in Gegenwart starker Basen, zum Beispiel Alkalimetallamiden.

Die stereoselektive Herstellung von Verbindungen der Formel B ist noch nicht bekannt. Es wurde nun überraschend gefunden, dass man stereospezifisch 2-Alkyl-3-heterocyclyl-1-propanole (Verbindungen der Formel B mit Y in der Bedeutung OH; im Folgenden als Verbindung der Formel I bezeichnet) in nur vier oder fünf Verfahrensstufen in hohen Ausbeuten herstellen kann: Wenn man, analog zu dem in der WO 02/02487 A1 beschrieben Verfahren, geeignet substituierte, ungesättigte Heterocyclyl-Aldehyde mit Carbonsäureestern zu 2-Alkyl-3-hydroxy-3-heterocyclyl carbonsäureestern kondensiert, fallen die diastereomerischen Produkte in hohen Ausbeuten an. Überraschend wurde gefunden, dass die erhaltenen Diastereomere, im Fall der 2-Alkyl-3-hydroxy-3-heterocyclyl carbonsäureester, mit Vorteil nicht getrennt werden, da nach Überführung der Hydroxygruppe in eine Abgangsgruppe gefolgt von baseninduzierter Elimination (E)-3-Heterocyclyl-2-methyl-acrylsäureester mit hoher Stereoselektivität gebildet werden. Die (E)-3-Heterocyclyl-2-methyl-acrylsäureester sind ein wichtiges Zwischenprodukt des Verfahrens. Ausgehend von diesen (E)-3-Heterocyclyl-2-methyl-acrylsäureestern lassen sich die 2-Alkyl-3-heterocyclyl-1-propanole durch drei Verfahrensvarianten erhalten:
1) Aus den rohen 3-Heterocyclyl-2-methyl-acrylsäureestern werden nach Verseifung und Kristallisation ausschliesslich (E)-3-Heterocyclyl-2-methyl-acrylsäuren in hohen Ausbeuten erhalten. Die (E)-3-Heterocyclyl-2-methyl-acrylsäuren können in Gegenwart bestimmter Katalysatoren zu praktisch enantiomerenreinen 2-Alkyl-3-heterocyclyl-1-propionsäuren hydriert werden, die durch Reduktion in 2-Alkyl-3-heterocyclyl-1-propanole der Formel I überführt werden können.
2) Aus den rohen 3-Heterocyclyl-2-methyl-acrylsäureestern werden nach Verseifung und Kristallisation ausschliesslich (E)-3-Heterocyclyl-2-methyl-acrylsäuren in hohen Ausbeuten erhalten. Die (E)-3-Heterocyclyl-2-methyl-acrylsäuren können zu Allylalkoholen reduziert werden; die erhaltenen Allylalkohole wiederum können in Gegenwart bestimmter Katalysatoren zu praktisch enantiomerenreinen 2-Alkyl-3-heterocyclyl-1-propanolen der Formel I hydriert werden.
3) Die (E)-3-Heterocyclyl-2-methyl-acrylsäureester können zu Allylalkoholen reduziert werden. Die erhaltenen Allylalkohole wiederum können in Gegenwart bestimmter Katalysatoren zu praktisch enantiomerenreinen 2-Alkyl-3-heterocyclyl-1-propanolen der Formel I hydriert werden.

Bei den Verfahrensvarianten 1) und 2) werden alle Verfahrensschritte bis zu den (E)-3-Heterocyclyl-2-methyl-acrylsäuren mit Vorteil ohne Reinigung der Zwischenprodukte durchgeführt, was für die Herstellung im industriellen Massstab einen erheblichen Vorteil (z.B. Kosteneinsparung) bedeutet. Die Verfahrensvariante 3) ist um eine Verfahrensstufe kürzer, was ebenfalls vorteilhaft für die Herstellung im industriellen Massstab ist.

Die auf diesen Wegen erhaltenen 2-Alkyl-3-heterocyclyl-1-propanole der untenstehenden Formel I können dann durch Halogenierung, in an sich bekannter Weise, beispielsweise nach dem von J. Maibaum in Tetrahedron Letters, Vol. 41 (2000), Seiten 10085-10089 beschriebenen Verfahren, in die Verbindungen der Formel B übergeführt werden.

Gegenstand der Erfindung ist ein Verfahren zur Herstellung von Verbindungen der Formel I, 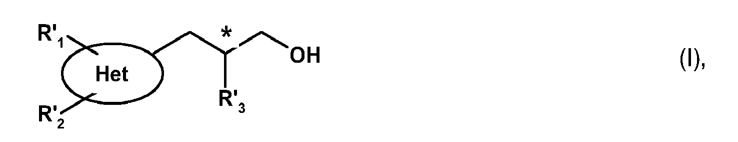 worin Het ein bicyclisches, über ein C-Atom an das Restmolekül geknüpftes, ungesättigtes Heterocyclyl bedeutet, wobei der nicht direkt an das Restmolekül gebundene Ring durch R'₁ und R'₂ substituiert ist, R'₁ und R'₂ unabhängig voneinander H, C₁-C₈-Alkyl, Halogen, Polyhalogen-C₁-C₈-alkoxy, Polyhalogen-C₁-C₈-alkyl, C₁-C₈-Alkoxy, C₁-C₈-Alkoxy-C₁-C₈-alkyl, oder C₁-C₈-Alkoxy-C₁-C₈-alkoxy darstellen, wobei R'₁ und R'₂ nicht gleichzeitig H bedeuten, R₃ C₁-C₈-Alkyl bedeutet und worin das Kohlenstoffatom, an welches der Rest R'₃ gebunden ist, entweder (R)- oder (S)-Konfiguration aufweist, wobei die (R)-Konfiguration bevorzugt ist, das dadurch gekennzeichnet ist, dass man
a) eine Verbindung der Formel II 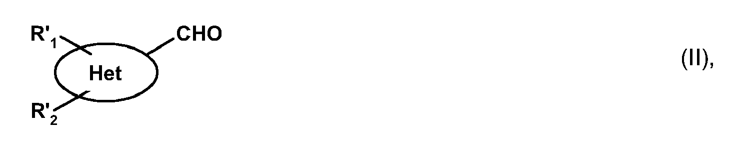 worin Het, R'₁ und R'₂ die zuvor angegebenen Bedeutungen haben, mit einer Verbindung der Formel III, 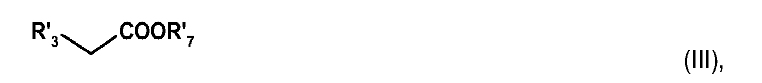 worin R'₃ die zuvor angegebene Bedeutung hat, zu einem Diastereomeren-Gemisch der Formel IV umsetzt, 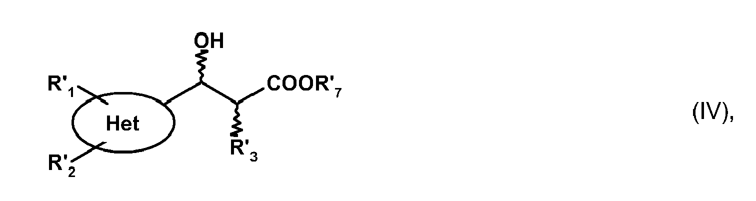 worin R'₇ für C₁-C₁₂-Alkyl, C₃-C₈-Cycloalkyl, Phenyl oder Benzyl steht,
b) die OH-Gruppe des Diastereomeren-Gemisches der Formel IV in eine Abgangsgruppe überführt und die eine Abgangsgruppe enthaltende Verbindung in Gegenwart einer starken Base zu einem Acrylsäureester der Formel V eliminiert,

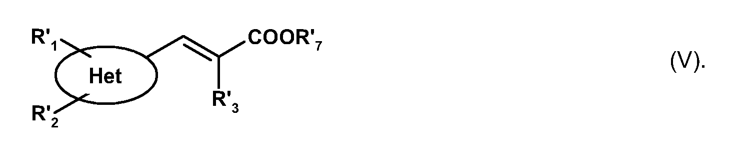

### Verfahrensvariante 1

Diese Verfahrensvariante ist dadurch gekennzeichnet, dass man
1c) den Acrylsäureester der Formel V durch Verseifung zu einer Verbindung der Formel VI umsetzt, 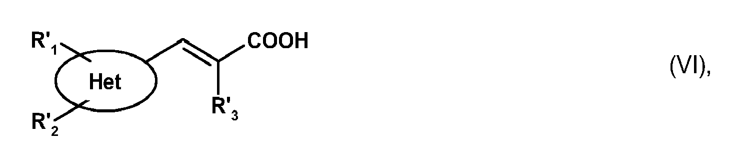
1d) die Säure der Formel VI in Gegenwart von Wasserstoff und katalytischen Mengen eines Metallkomplexes als asymmetrischer Hydrierkatalysator, der Metalle aus der Gruppe Ruthenium, Rhodium oder Iridium enthält, an die chirale, zweizähnige Liganden gebunden sind, zu einer Verbindung der Formel VII hydriert, 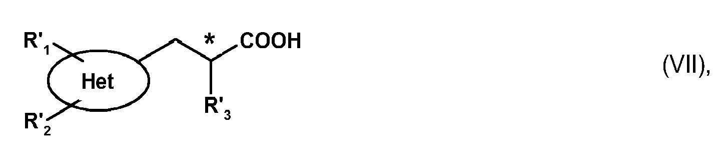 und
1e) die Säure der Formel VII zu einer Verbindung der Formel I reduziert.

### Verfahrensvariante 2

Diese Verfahrensvariante ist dadurch gekennzeichnet, dass man
2c) den Acrylsäureester der Formel V durch Verseifung zu einer Verbindung der Formel VI umsetzt, 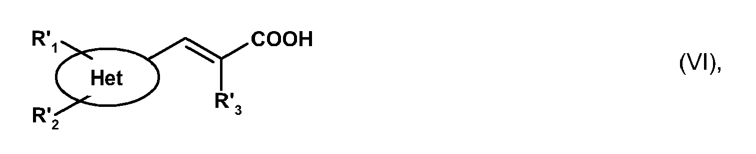
2d) die Säure der Formel VI, zu einer Verbindung der Formel VIII reduziert 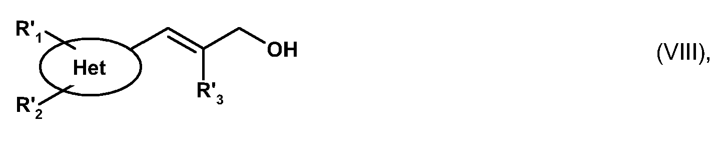 und
2e) den Alkohol der Formel VIII in Gegenwart von Wasserstoff und katalytischen Mengen eines Metallkomplexes als asymmetrischer Hydrierkatalysator, der Metalle aus der Gruppe Ruthenium, Rhodium oder Iridium enthält, an die chirale, zweizähnige Liganden gebunden sind, zu einer Verbindung der Formel I hydriert.

### Verfahrensvariante 3

Diese Verfahrensvariante ist dadurch gekennzeichnet, dass man
3c) den Acrylsäureester der Formel V zu einer Verbindung der Formel VIII reduziert 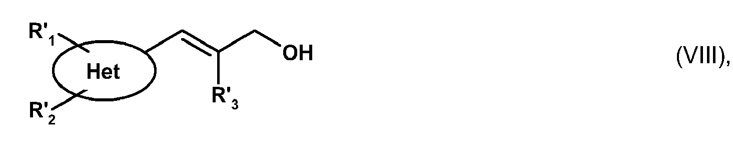 und
3d) den Alkohol der Formel VIII in Gegenwart von Wasserstoff und katalytischen Mengen eines Metallkomplexes als asymmetrischer Hydrierkatalysator, der Metalle aus der Gruppe Ruthenium, Rhodium oder Iridium enthält, an die chirale zweizähnige Liganden gebunden sind, zu einer Verbindung der Formel I hydriert.

R'₁ und R'₂ können als C₁-C₈-Alkyl linear oder verzweigt sein und bevorzugt 1 bis 4 C-Atome enthalten. Beispiele sind Methyl, Ethyl, n- und i-Propyl, n-, i- und t-Butyl, Pentyl und Hexyl.

R'₁ und R'₂ können als Polyhalogen-C₁-C₈-alkyl linear oder verzweigt sein und bevorzugt 1 bis 4, besonders bevorzugt 1 oder 2 C-Atome enthalten. Beispiele sind Fluormethyl, Difluormethyl, Trifluormethyl, Chlormethyl, Dichlormethyl, Trichlormethyl, 2-Chlorethyl und 2,2,2-Trifluorethyl.

R'₁ und R'₂ können als Polyhalogen-C₁-C₈-alkoxy linear oder verzweigt sein und bevorzugt 1 bis 4, besonders bevorzugt 1 oder 2 C-Atome enthalten. Beispiele sind Fluormethoxy, Difluormethoxy, Trifluormethoxy, Chlormethoxy, Dichlormethoxy, Trichlormethoxy, 2-Chlorethoxy und 2,2,2-Trifluorethoxy.

R'₁ und R'₂ können als Halogen, einschliesslich Halogen in Polyhalogen-C₁-C₈-alkyl und Polyhalogen-C₁-C₈-alkoxy, F, Cl oder Br bedeuten, wobei F und Cl bevorzugt sind.

R'₁, R'₂ und R'₅ können als C₁-C₈-Alkoxy linear oder verzweigt sein und bevorzugt 1 bis 4 C-Atome enthalten. Beispiele sind Methoxy, Ethoxy, n- und i-Propoxy, n-, i- und t-Butoxy, Pentoxy und Hexoxy.

R'₁ und R'₂ können als C₁-C₈-Alkoxy-C₁-C₈-alkyl linear oder verzweigt sein. Die Alkoxygruppe enthält bevorzugt 1 bis 4 und besonders 1 oder 2 C-Atome, und die Alkylgruppe enthält bevorzugt 1 bis 4 C-Atome. Beispiele sind Methoxymethyl, 1-Methoxyeth-2-yl, 1-Methoxyprop-3-yl, 1-Methoxybut-4-yl, Methoxypentyl, Methoxyhexyl, Ethoxymethyl, 1-Ethoxyeth-2-yl, 1-Ethoxyprop-3-yl, 1-Ethoxybut-4-yl, Ethoxypentyl, Ethoxyhexyl, Propoxymethyl, Butoxymethyl, 1-Propoxyeth-2-yl und 1-Butoxyeth-2-yl.

R'₁ und R'₂ können als C₁-C₈-Alkoxy-C₁-C₈-alkoxy linear oder verzweigt sein. Die Alkoxygruppe enthält bevorzugt 1 bis 4 und besonders 1 oder 2 C-Atome, und die Alkoxygruppe enthält bevorzugt 1 bis 4 C-Atome. Beispiele sind Methoxymethoxy, 2-Methoxyethoxy, 3-Methoxypropoxy, 4-Methoxybutoxy, Methoxypentoxy, Methoxyhexoxy, Ethoxymethoxy, 2-Ethoxyethoxy, 3-Ethoxypropoxy, 4-Ethoxybutoxy, Ethoxypentoxy, Ethoxyhexoxy, Propoxymethoxy, Butoxymethoxy, 2-Propoxyethoxy und 2-Butoxyethoxy.

In einer bevorzugten Ausführungsform bedeutet R'₁ Methoxy- oder Ethoxy-C₁-C₄-alkyl, und R'₂ stellt bevorzugt Methyl, Ethyl, Methoxy oder Ethoxy dar. Ganz besonders bevorzugt sind Verbindungen der Formel I, worin R'₁ 3-Methoxy-propyl oder 4-Methoxy-butyl und R'₂ Methyl oder Methoxy bedeuten.

R'₃, R'₄, R'₅ und R'₆ können als C₁-C₈-Alkyl linear oder verzweigt sein und bevorzugt 1 bis 4 C-Atome enthalten. Beispiele sind Methyl, Ethyl, n- und i-Propyl, n-, i- und t-Butyl, Pentyl und Hexyl. In einer bevorzugten Ausführungsform stellt in den Verbindungen der Formel I R'₃ Isopropyl dar und das Kohlenstoffatom, an welches der Rest R'₃ gebunden ist, weist (R)-Konfiguration auf.

Het kann als bicyclisches, über ein C-Atom an das Restmolekül geknüpftes, ungesättigtes Heterocyclyl bicyclische, ungesättigte heterocyclische Reste mit 1 bis 4 Stickstoff- und/oder 1 oder 2 Schwefel- oder Sauerstoffatomen umfassen, wobei Reste mit einem oder 2 Stickstoffatomen bevorzugt sind. Bevorzugte Bicyclen bestehen aus jeweils 5- und/oder 6-gliedrigen Ringen. Beispiele für Het sind Benzothiazolyl, Chinazolinyl, Chinolyl, Chinoxalinyl, Isochinolyl, Benzo[b]thienyl, Isobenzofuranyl, Benzoimidazolyl, Indolyl, Dihydrobenzofuranyl, Tetrahydro-chinoxalinyl, 3,4-Dihydro-2H-benzo[1,4]oxazinyl, 1 H-Pyrrolizinyl, Phthalazinyl, Dihydro-2H-benzo[1,4]thiazinyl, 1 H-Pyrrolo[2,3-b]pyridyl, Imidazo[1,5-a]pyridyl, Benzooxazolyl, 2,3-Dihydroindolyl, Indazolyl oder Benzofuranyl. Het bedeutet besonders bevorzugt 1 H-Indol-6-yl, Imidazo[1,5-a]pyridin-6-yl oder 1 H-Indazol-6-yl.

Besonders bevorzugt sind Verbindungen der Formel I, worin mit R'₁ und R'₂ substituiertes Het 1-(3-Methoxy-propyl)-3-methyl-1H-indol-6-yl, 3-(3-Methoxy-propyl)-1 -methyl-imidazo[1,5-a]pyridin-6-yl oder 1-(3-Methoxy-propyl)-3-methyl-1 H-indazol-6-yl und R'₃ Isopropyl darstellen.

R'₇ kann als C₃-C₈-Cycloalkyl Cyclopropyl, Cyclobutyl, Cyclopentyl, Cyclohexyl, Cycloheptyl oder Cyclooctyl bedeuten.

R'₇ bedeutet bevorzugt C₁-C₆- und besonders bevorzugt C₁-C₄-Alkyl; einige Beispiele sind Methyl, Ethyl, n-Propyl und n-Butyl.

Die in der Verfahrensstufe a) verwendeten Ausgangsverbindungen der Formeln II und III sind bekannt oder können analog zu bekannten Verfahren hergestellt werden. Verbindungen der Formel II werden nach an sich bekannter Weise aus den in der WO 2005/090305 A1 beschrieben bicyclischen, ungesättigten Heterocyclyl-Bromiden über Halogen-Metall-Austausch und anschliessender Reaktion mit N,N-Dimethylformamid hergestellt. Die Reaktion der Verfahrensstufe a) wird vorteilhaft bei niedrigen Temperaturen, zum Beispiel -40 bis 0°C, in Gegenwart wenigstens äquivalenter Mengen einer starken Base durchgeführt.

Die Reaktion wird ferner zweckmässig in einem Lösungsmittel durchgeführt, wobei Ether wie zum Beispiel Diethylether, Tetrahydrofuran und Dioxan besonders geeignet sind. Geeignete starke Basen sind insbesondere Alkalimetallalkoholate und -sekundäramide, zum Beispiel Lithium-düsopropylamid.

Das Gemisch der beiden Diastereomeren der Formel IV wird in nahezu quantitativer Ausbeute erhalten. Vorteilhaft wird das Diastereomeren-Gemisch ohne Reinigung in der nächsten Verfahrensstufe eingesetzt.

Die Überführung der OH-Gruppe in eine Abgangsgruppe in der Verfahrensstufe b) ist an sich bekannt. Besonders geeignet ist die Umsetzung mit Carbonsäuren oder Sulfonsäuren, beziehungsweise deren Säurechloride oder Anhydriden (Acylierung). Einige Beispiele für Carbon- oder Sulfonsäuren sind Ameisensäure, Essigsäure, Propionsäure, Benzoesäure, Benzolsulfonsäure, Toluolsulfonsäure, Methylsulfonsäure und Trifluormethylsulfonsäure. Die Verwendung von Essigsäureanhydrid in Gegenwart katalytischer Mengen von 4-Dimethylaminopyridin hat sich besonders bewährt. Die Elimination wird zweckmässig in Gegenwart von starken Basen vorgenommen, wobei Alkalimetallalkoholate wie Kaliumtertiärbutylat besonders geeignet sind. Die Gegenwart von Lösungsmitteln wie Ethern ist zweckmässig. Die Reaktion wird vorteilhaft bei niedrigen Temperaturen, zum Beispiel 0°C bis 40°C, durchgeführt. Vorteilhaft wird die Eliminationsreaktion direkt in der Reaktionsmischung der Verfahrensstufe a) durchgeführt. Die Elimination führt überraschend selektiv zu den gewünschten E-Isomeren der Acrylsäureester der Formel V.

Die Verseifung der Acrylsäureester der Formel V in den Verfahrenssstufen 1 c) bzw. 2c) wird vorteilhaft direkt nach Erreichen des vollständigen Umsatzes der Elimination (Verfahrensstufe b) und nach Einengen des Lösungsmittels, durch Zugabe von, beispielsweise, Kalilauge und Rühren bei Temperaturen zwischen 80°C und 100°C vorgenommen. Die erhaltenen Säuren der Formel VI sind hochkristallin und können daher in einfacher Weise ohne grosse Verluste mittels Extraktion und Kristallisation isoliert werden. Die Ausbeuten liegen über 60%. Es werden überraschend ausschliesslich die gewünschten E-Isomere erhalten.

Asymmetrischen Hydrierungen in Analogie zur Verfahrensstufe 1d) von α,β-ungesättigten Carbonsäuren der Formel VI und zu den Verfahrensstufen 2e) bzw. 3d) von α,β-ungesättigten Alkoholen der Formel VIII mit homogenen, asymmetrischen Hydrierkatalysatoren sind an sich bekannt und zum Beispiel von J. M. Brown in E. Jacobsen, A. Pfaltz, H. Yamamoto (Eds.) in Comprehensive Asymmetric Catalysis I bis III, Springer Verlag, 1999, Seiten 121-182 und von X. Zhang in Chemical Reviews, Vol 103 (2003), Seiten 3029-3069 beschrieben. Besonders wirksam sind Ruthenium-, Rhodium- und lridiumkatalysatoren.

Asymmetrische Hydrierungen von α,β-ungesättigten Carbonsäuren der Formel VI können allgemein bevorzugt unter Verwendung von Ruthenium-, oder Rhodiumkatalysatoren, wie zum Beispiel von J. M. Brown in E. Jacobsen, A. Pfaltz, H. Yamamoto (Eds.) in Comprehensive Asymmetric Catalysis I bis III, Springer Verlag, 1999, Seiten 163-166, W. Weissensteiner und F. Spindler im Advanced Synthesis and Catalysis Vol. 345 (2003), Seiten 160-164 und von T. Yamagishi im Journal of the Chemical Society, Perkin Transactions 1, (1997) Seiten 1869-1873 beschrieben, durchgeführt werden.

Als Liganden für Rhodium und Ruthenium werden häufig chirale ditertiäre Bis-phosphine verwendet. Solche chiralen ditertiären Bis-phosphine sind zum Beispiel von X. Zhang in Chemical Reviews, Vol 103 (2003), Seiten 3029-3069 beschrieben.

Liganden mit einem Ferrocenylgerüst sind im Allgemeinen besonders für die asymmetrische Hydrierung von α,β-ungesättigten Carbonsäuren geeignet. Beispiele sind von F. Spindler in Tetrahedron: Asymmetry, Vol. 15 (2004), Seiten 2299-2306 beschrieben. Beispiele sind Liganden der Klassen Walphos, Josiphos, Mandyphos und Taniaphos. Liganden dieser Klassen sind zum Beispiel von X. Zhang in Chemical Reviews, Vol 103 (2003), Seiten 3029-3069, sowie von P. Knochel in Chemistry, a European Journal Vol. 8 (2002), Seiten 843-852, von H.-U. Blaser in Topics in Catalysis, Vol 19 (2002), Seiten 3-16 und von F. Spindler in Tetrahedron: Asymmetry, Vol. 15 (2004), Seiten 2299-2306 beschrieben.

Als Liganden für Iridium werden häufig chirale Phosphin-oxazolin-Liganden oder Phosphinitoxazolin-Liganden verwendet. Solche chiralen Phosphin-oxazolin-Liganden oder Phosphinitoxazolin-Liganden sind zum Beispiel von A. Pfaltz im Advanced Synthesis and Catalysis Vol. 345 (2003), Seiten 33-43 beschrieben.

Gute optische Ausbeuten werden insbesondere mit Metallkomplexen der Formeln IX oder IXa erzielt,

[LM¹X¹X²] (IX),

[LM¹X¹]⁺E⁻ (IXa),

worin
M¹ Rhodium oder Iridium bedeutet;
X¹ für zwei Olefine oder ein Dien steht;
X² Cl, Br oder I bedeutet;
E⁻ das Anion einer Sauerstoffsäure oder Komplexsäure darstellt; und
L für einen chiralen Liganden aus der Gruppe der Ferrocen-1,1'-diphosphine, die in 1-Stellung eine Ferrocen-substituierte, sekundäre Phosphingruppe und in 1'-Stellung eine sekundäre Phosphingruppe aufweisen, steht.

Bei X¹ in der Bedeutung als Olefin kann es sich um C₂-C₁₂-, bevorzugt C₂-C₆- und besonders bevorzugt C₂-C₄-Olefine handeln. Beispiele sind Propen, Buten und besonders Ethylen. Das Dien kann 5 bis 12 und bevorzugt 5 bis 8 C-Atome enthalten und es kann sich um offenkettige, cyclische oder polycyclische Diene handeln. Die beiden Olefingruppen des Diens sind bevorzugt durch ein oder zwei CH₂-Gruppen verbunden. Beispiele sind 1,3-Pentadien, Cyclopentadien, 1,5-Hexadien, 1,4-Cyclohexadien, 1,4- oder 1,5-Heptadien, 1,4- oder 1,5-Cycloheptadien, 1,4- oder 1,5-Octadien, 1,4- oder 1,5-Cyclooctadien und Norbornadien. Bevorzugt stellt X¹ zwei Ethylen oder 1,5- Hexadien, 1,5-Cyclooctadien oder Norbornadien dar.

In Formel IX steht X² bevorzugt für Cl oder Br. Beispiele für E⁻ sind ClO₄⁻, CF₃SO₃⁻, CH₃SO₃⁻, HSO₄⁻, BF₄⁻, B(Phenyl)₄⁻, BARF (B(3,5-bis(Trifluormethyl)phenyl)₄⁻, PF₆⁻, SbCl₆⁻, AsF₆⁻ oder SbF₆⁻.

Es wurde nun überraschend gefunden, dass Liganden vom Typ der Ferrocen-1,1'-diphosphine, die in 1-Stellung eine Ferrocen-substituierte, sekundäre Phosphingruppe und in 1'-Stellung eine sekundäre Phosphingruppe aufweisen, besonders für die asymmetrische Hydrierung von α,β-ungesättigten Carbonsäuren geeignet sind. Mit diesen Liganden in den Metallkomplexen der Formeln IX und IXa können hohe optische Ausbeuten erreicht werden, was für die Herstellung im industriellen Massstab einen erheblichen Vorteil (beispielsweise Kosteneinsparung). Überraschend ist in diesem Zusammenhang auch die Verwendung von Iridium-Komplexen für die asymmetrische Hydrierung von α,β-ungesättigten Carbonsäuren, was für die Herstellung im industriellen Massstab einen weiteren, erheblichen Vorteil (beispielsweise weitere Kosteneinsparung im Vergleich zur Verwendung z.B. von teureren Rhodiumkomplexen) bedeutet. In den Verfahrensstufen 1d), ausgehend von α,β-ungesättigten Carbonsäuren der Formel VI können daher zum Beispiel Metallkomplexe der Formeln IX und IXa eingesetzt werden, deren Liganden zu den Ferrocen-1,1'-diphosphinen, die in 1-Stellung eine Ferrocen-substituierte, sekundäre Phosphingruppe und in 1'-Stellung eine sekundäre Phosphingruppe aufweisen, gehören.

Im Rahmen der vorliegenden Erfindung sind Ferrocen-1,1'-diphosphine, die in 1-Stellung eine Ferrocen-substituierte, sekundäre Phosphingruppe und in 1'-Stellung eine sekundäre Phosphingruppeaufweisen, Verbindungen der Formel X in Form von enantiomerenreinen Diastereomeren oder einem Gemisch von Diastereomeren, 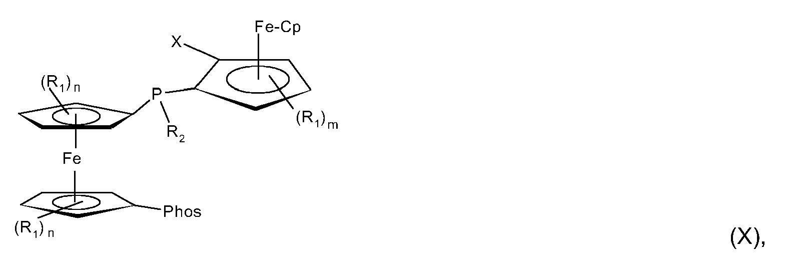 worin
die R₁ gleich oder verschieden sind und C₁-C₄-Alkyl bedeuten;
m für 0 steht oder eine ganze Zahl von 1 bis 3 darstellt;
n für 0 steht oder eine ganze Zahl von 1 bis 4 darstellt;
R₂ einen Kohlenwasserstoffrest oder C-gebundenen Heterokohlenwasserstoffrest bedeutet; Cp für unsubstituiertes oder mit C₁-C₄-Alkyl substituiertes Cyclopentadienyl steht;
X eine C-gebundene, Metalle von Metallierungsreagenzien in die Orthostellung dirigierende, chirale Gruppe darstellt; und
Phos einen P-gebundenen P(III)-Substituenten bedeutet.

Phos stellt als P-gebundener P(III)-Substituent besonders bevorzugt eine sekundäre Phosphingruppe dar.

Bei R₁ als Alkyl kann es sich zum Beispiel um Methyl, Ethyl, n- oder i-Propyl, n-, i- oder t-Butyl handeln, wobei Methyl bevorzugt ist. Bevorzugt stehen m und n für 0 (und R₁ ist damit ein Wasserstoffatom).

Die Kohlenwasserstoffreste R₂ können unsubstituiert oder substituiert sein und und/oder Heteroatome ausgewählt aus der Gruppe O, S, -N= oder N(C₁-C₄-Alkyl) enthalten. Sie können 1 bis 22, bevorzugt 1 bis 18, besonders bevorzugt 1 bis 12 und insbesondere bevorzugt 1 bis 8 C-Atome sowie 1 bis 4 und bevorzugt 1 oder 2 der genannten Heteroatome enthalten. Bei R₂ kann es sich um Reste, ausgewählt aus der Gruppe lineares oder verzweigtes C₁-C₁₂-Alkyl; unsubstituiertes oder mit C₁-C₆-Alkyl oder C₁-C₆-Alkoxy substituiertes C₄-C₁₂-Cycloalkyl oder C₄-C₁₂-Cycloalkyl-CH₂-; C₆-C₁₄-Aryl; C₄-C₁₂-Heteroaryl; C₇-C₁₄-Aralkyl; C₄-C₁₂-Heteroaralkyl; oder mit Halogen (Fluor, Chlor oder Brom), C₁-C₆-Alkyl, Trifluormethyl, C₁-C₆-Alkoxy, Trifluormethoxy, (C₆H₅)₃Si, (C₁-C₁₂-Alkyl)₃Si, oder Sekundäramino substituiertes C₆-C₁₄-Aryl, C₄-C₁₂-Heteroaryl, C₇-C₁₄-Aralkyl oder C₄-C₁₂-Heteroaralkyl handeln. Heteroaryl und Heteroaralkyl enthalten bevorzugt Heteroatome ausgewählt aus der Gruppe O, S und -N=.

Beispiele für R₂ als Alkyl, das bevorzugt 1 bis 6 C-Atome enthält, sind Methyl, Ethyl, n-Propyl, i-Propyl, n-Butyl, i-Butyl, t-Butyl, und die Isomeren von Pentyl und Hexyl. Beispiele für R₂ als gegebenenfalls mit Alkyl substituiertes Cycloalkyl sind Cyclopentyl, Cyclohexyl, Methyl-und Ethylcyclohexyl, Dimethylcyclohexyl, Cycloheptyl, Cyclooctyl, Norbornyl und Adamantyl. Beispiele für R₂ als gegebenenfalls mit Alkyl, und Alkoxy substituiertes C₅-C₁₂-Cycloalkyl-CH₂- sind Cyclopentylmethyl, Cyclohexylmethyl, Cyclooctylmethyl, Methylcyclohexylmethyl und Dimethylcyclohexylmethyl. Beispiele für R₂ als Aryl und Aralkyl sind Phenyl, Naphthyl, Anthracenyl, Fluorenyl, Benzyl und Naphthylmethyl. Beispiele für R₂ als Heteroaryl und Heteroaralkyl sind Furyl, Thiophenyl, N-Methylpyrrolidinyl, Pyridyl, Benzofuranyl, Benzothiophenyl, Chinonlinyl, Furylmethyl, Thiophenylmethyl und Pyridylmethyl. Beispiele für R₂ als substituiertes Aryl, Aralkyl, Heteroaryl und Heteroaralkyl sind Phenyl, Naphthyl, Benzyl, Naphthylmethyl, Phenylethyl, Furyl, Thiophenyl, Benzofuryl und Benzothiophenyl, die mit 1 bis 3 Resten ausgewählt aus der Gruppe Methyl, Ethyl, n- und i-Propyl, n-, i- und t-Butyl, Ethoxy, Methoxy, Trifluormethyl, Trifluormethoxy, Fluor oder Chlor substituiert sind. Einige bevorzugte Beispiele sind 2-, 3- oder 4-Methylphenyl, 2,4- oder 3,5-Dimethylphenyl, 3,4,5-Trimethylphenyl, 4-Ethylphenyl, 2- oder 4-Methylbenzyl, 2-, 3- oder 4-Methoxyphenyl, 2,4- oder 3,5-Dimethoxyphenyl, 3,4,5-Trimethoxyphenyl, 2-, 3- oder 4-Trifluormethylphenyl, 2,4- oder 3,5-Di(trifluormethyl)phenyl, Tris-trifluormethylphenyl, 2- oder 4-Trifluormethoxyphenyl, 3,5-Bis-trifluormethoxyphenyl, 2- oder 4-Fluor-phenyl, 2- oder 4-Chlorphenyl und 3,5-Dimethyl-4-methoxyphenyl.

In einer besonders bevorzugten Ausführungsform bedeutet R₂ C₁-C₆-Alkyl, C₅-C₈-Cycloalkyl, C₇-C₈-Bicycloalkyl, o-Furyl, Phenyl, Naphthyl, 2-(C₁-C₆-Alkyl)C₆H₄, 3-(C₁-C₆-Alkyl)C₆H₄, 4-(C₁-C₆-Alkyl)C₆H₄, 2-(C₁-C₆-Alkoxy)C₆H₄, 3-(C₁-C₆-Alkoxy)C₆H₄, 4-(C₁-C₆-Alkoxy)C₆H₄, 2-(Trifluormethyl)C₆H₄, 3-(Trifluormethyl)C₆H₄, 4-(Trifluormethyl)C₆H₄, 3,5-Bis(trifluormethyl)C₆H₃, -3,5-Bis(C₁-C₆-Alkyl)₂C₆H₃, 3,5-Bis(C₁-C₆-Alkoxy)₂C₆H₃, und 3,5-Bis(C₁-C₆-Alkyl)₂-4-(C₁-C₆-Alkoxy)C₆H₂.

Bei der orthodirigierenden, chiralen Gruppe X ist das chirale Atom vorzugsweise in 1-, 2-oder 3-Stellung zur Bindung Cyclopentadienyl-X gebunden. Bei der Gruppe X kann es sich um offenkettige Reste oder zyklische Reste handeln, wobei die Atome ausgewählt sind aus der Gruppe H, C, O, S und N.

Die Gruppe X kann zum Beispiel der Formel -HC*R₅R₆ (mit * wird das asymmetrische Atom gekennzeichnet) entsprechen, worin R₅ C₁-C₈-Alkyl, C₅-C₈-Cycloalkyl (Cyclohexyl), C₆-C₁₀-Aryl (Phenyl), C₇-C₁₂-Aralkyl (Benzyl) oder C₇-C₁₂-Alkaralkyl (Methylbenzyl) steht, R₆ für -OR₇ oder-NR₈R₉ steht, R₇ C₁-C₈-Alkyl, C₅-C₈-Cycloalkyl, Phenyl oder Benzyl bedeutet, und R₈ und R₉ gleich oder verschieden sind und C₁-C₈-Alkyl, C₅-C₈-Cycloalkyl, Phenyl oder Benzyl darstellen oder R₈ und R₉ zusammen mit dem N-Atom einen fünf- bis achtgliedrigen Ring bilden. R₅ ist bevorzugt C₁-C₄-Alkyl wie zum Beispiel Methyl, Ethyl, n-Propyl und Phenyl. R₇ bedeutet bevorzugt C₁-C₄-Alkyl wie zum Beispiel Methyl, Ethyl, n-Propyl und n- oder i-Butyl. R₈ und R₉ bedeuten bevorzugt gleiche Reste und stellen bevorzugt C₁-C₄-Alkyl wie zum Beispiel Methyl, Ethyl, n-Propyl, i-Propyl und n- oder i-Butyl und zusammen Teramethylen, Pentamethylen oder 3-Oxa-1,5-pentylen dar. Besonders bevorzugte Gruppen der Formel -HCR₅R₆ sind 1-Methoxy-eth-1-yl, 1-Dimethylamino-eth-1-yl und 1-(Dimethylamino)-1-phenyl-methyl.

X stellt besonders bevorzugt eine Gruppe -CHR₅-NR₈R₉ dar, worin R₅ C₁-C₄-Alkyl, C₅-C₆-Cycloalkyl, Phenyl, C₁-C₄-Alkylphenyl oder C₁-C₄-Alkylbenzyl steht, und R₈ und R₉ gleich sind und C₁-C₄-Alkyl und bevorzugt Methyl oder Ethyl darstellen.

Phos kann als P-gebundener P(III)-Substituent eine sekundäre Phosphingruppe sein, die gleiche oder verschiedene Kohlenwasserstoffreste enthält, oder in denen der Kohlenwasserstoffrest zusammen mit dem P-Atom einen 4- bis 8-gliedrigen Ring bildet. Bevorzugt enthält die sekundäre Phosphingruppe gleiche Kohlenwasserstoffreste. Die Kohlenwasserstoffreste können unsubstituiert oder substituiert sein und und/oder Heteroatome ausgewählt aus der Gruppe O, S, -N= oder N(C₁-C₄-Alkyl) enthalten. Sie können 1 bis 22, bevorzugt 1 bis 18, besonders bevorzugt 1 bis 12 und insbesondere bevorzugt 1 bis 8 C-Atome 1 bis 4 und bevorzugt 1 oder 2 der genannten Heteroatome enthalten. Es kann sich um Reste, ausgewählt aus der Gruppe lineares oder verzweigtes C₁-C₁₂-Alkyl; unsubstituiertes oder mit C₁-C₆-Alkyl oder C₁-C₆-Alkoxy substituiertes C₅-C₁₂-Cycloalkyl oder C₅-C₁₂-Cycloalkyl-CH₂-; C₆-C₁₄-Aryl; C₄-C₁₂-Heteroaryl; C₇-C₁₄-Aralkyl; C₄-C₁₂-Heteroaralkyl; oder mit Halogen (Fluor, Chlor oder Brom), C₁-C₆-Alkyl, Trifluormethyl, C₁-C₆-Alkoxy, Trifluormethoxy, (C₆H₅)₃Si, (C₁-C₁₂-Alkyl)₃Si, oder Sekundäramino substituiertes C₆-C₁₄-Aryl, C₄-C₁₂-Heteroaryl, C₇-C₁₄-Aralkyl oder C₄-C₁₂-Heteroaralkyl handeln. Heteroaryl und Heteroaralkyl enthalten bevorzugt Heteroatome ausgewählt aus der Gruppe O, S und -N=.

Beispiele für Kohlenwasserstoffreste als Alkyl, das bevorzugt 1 bis 6 C-Atome enthält, sind Methyl, Ethyl, n-Propyl, i-Propyl, n-Butyl, i-Butyl, t-Butyl, und die Isomeren von Pentyl und Hexyl. Beispiele für Kohlenwasserstoffreste als gegebenenfalls mit Alkyl substituiertes Cycloalkyl sind Cyclopentyl, Cyclohexyl, Methyl- und Ethylcyclohexyl, Dimethylcyclohexyl, Cycloheptyl, Cyclooctyl, Norbornyl und Adamantyl. Beispiele für Kohlenwasserstoffreste als gegebenenfalls mit Alkyl, und Alkoxy substituiertes C₅-C₁₂-Cycloalkyl-CH₂- sind Cyclopentylmethyl, Cyclohexylmethyl, Cyclooctylmethyl, Methylcyclohexylmethyl und Dimethylcyclohexylmethyl. Beispiele für Kohlenwasserstoffreste als Aryl und Aralkyl sind Phenyl, Naphthyl, Anthracenyl, Fluorenyl, Benzyl und Naphthylmethyl. Beispiele für Kohlenwasserstoffreste als Heteroaryl und Heteroaralkyl sind Furyl, Thiophenyl, N-Methylpyrrolidinyl, Pyridyl, Benzofuranyl, Benzothiophenyl, Chinonlinyl, Furylmethyl, Thiophenylmethyl und Pyridylmethyl. Beispiele für Kohlenwasserstoffreste als substituiertes Aryl, Aralkyl, Heteroaryl und Heteroaralkyl sind Phenyl, Naphthyl, Benzyl, Naphthylmethyl, Phenylethyl, Furyl, Thiophenyl, Benzofuryl und Benzothiophenyl, die mit 1 bis 3 Resten ausgewählt aus der Gruppe Methyl, Ethyl, n-und i-Propyl, n-, i- und t-Butyl, Ethoxy, Methoxy, Trifluormethyl, Trifluormethoxy, Fluor oder Chlor substituiert sind. Einige bevorzugte Beispiele sind 2-, 3- oder 4-Methylphenyl, 2,4- oder 3,5-Dimethylphenyl, 3,4,5-Trimethylphenyl, 4-Ethylphenyl, 2- oder 4-Methylbenzyl, 2-, 3-oder 4-Methoxyphenyl, 2,4- oder 3,5-Dimethoxyphenyl, 3,4,5-Trimethoxyphenyl, 2-, 3- oder 4-Trifluormethylphenyl, 2,4- oder 3,5-Di(trifluormethyl)phenyl, Tris-trifluormethylphenyl, 2-oder 4-Trifluormethoxyphenyl, 3,5-Bis-trifluormethoxyphenyl, 2- oder 4-Fluor-phenyl, 2- oder 4-Chlorphenyl und 3,5-Dimethyl-4-methoxyphenyl.

In einer besonders bevorzugten Ausführungsform bedeutet der Kohlenwasserstoffrest C₁-C₆-Alkyl, C₅-C₈-Cycloalkyl, C₇-C₈-Bicycloalkyl, o-Furyl, Phenyl, Naphthyl, 2-(C₁-C₆-Alkyl)C₆H₄, 3-(C₁-C₆-Alkyl)C₆H₄, 4-(C₁-C₆-Alkyl)C₆H₄, 2-(C₁-C₆-Alkoxy)C₆H₄, 3-(C₁-C₆-Alkoxy)C₆H₄, 4-(C₁-C₆-Alkoxy)C₆H₄, 2-(Trifluormethyl)C₆H₄, 3-(Trifluormethyl)C₆H₄, 4-(Trifluormethyl)C₆H₄, 3,5-Bis(trifluormethyl)C₆H₃, -3,5-Bis(C₁-C₆-Alkyl)₂C₆H₃, 3,5-Bis(C₁-C₆-Alkoxy)₂C₆H₃, und 3,5-Bis(C₁-C₆-Alkyl)₂-4-(C₁-C₆-Alkoxy)C₆H₂.

Die Sekundärphosphinogruppe entspricht bevorzugt der Formel -PR₃R₄, worin R₃ und R₄ unabhängig voneinander einen Kohlenwasserstoffrest mit 1 bis 18 C-Atomen darstellen, der unsubstituiert oder substituiert ist mit C₁-C₆-Alkyl, Trifluormethyl, C₁-C₆-Alkoxy, Trifluormethoxy, (C₁-C₄-Alkyl)₂amino, (C₆H₅)₃Si, (C₁-C₁₂-Alkyl)₃Si, Halogen, und/oder Heteroatome O enthält.

Bevorzugt sind R₃ und R₄ Reste, ausgewählt aus der Gruppe lineares oder verzweigtes C₁-C₆-Alkyl, unsubstituiertes oder mit ein bis drei C₁-C₄-Alkyl oder C₁-C₄-Alkoxy substituiertes Cyclopentyl oder Cyclohexyl, Furyl, unsubstituiertes oder mit ein bis drei C₁-C₄-Alkyl oder C₁-C₄-Alkoxy substituiertes Benzyl, und insbesondere unsubstituiertes oder mit ein bis drei F, Cl, C₁-C₄-Alkyl, C₁-C₄-Alkoxy, C₁-C₄-Fluoralkyl oder C₁-C₄-Fluoralkoxy substituiertes Phenyl oder Naphthyl.

Besonders bevorzugt bedeuten R₃ und R₄ Reste, ausgewählt aus der Gruppe C₃-C₈-Alkyl, Cyclopentyl, Cyclohexyl, Furyl, Naphthyl, und unsubstituiertes oder mit ein bis drei F, Cl, C₁-C₄-Alkyl, C₁-C₄-Alkoxy und/oder C₁-C₄-Fluoralkyl substituiertes Phenyl.

Bei der sekundären Phosphingruppe Phos kann es sich um zyklisches Sekundärphosphino handeln, zum Beispiel solche der Formeln 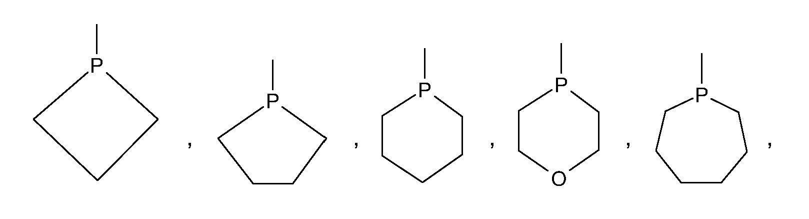 die unsubstituiert oder ein- oder mehrfach substituiert sind mit C₁-C₈-Alkyl, C₄-C₈-Cycloalkyl, C₁-C₆-Alkoxy, C₁-C₄-Alkoxy-C₁-C₄-Alkyl, Phenyl, C₁-C₄-Alkyl- oder C₁-C₄-Alkoxyphenyl, Benzyl, C₁-C₄-Alkyl- oder C₁-C₄-Alkoxybenzyl, Benzyloxy, C₁-C₄-Alkyl- oder C₁-C₄-Alkoxybenzyloxy, oder C₁-C₄-Alkyliden-dioxyl.

Die Substituenten können in einer oder beiden α-Stellungen zum P-Atom gebunden sein, um chirale C-Atome einzuführen. Bei den Substituenten in einer oder beiden α-Stellungen handelt es sich bevorzugt um C₁-C₄-Alkyl oder Benzyl, zum Beispiel um Methyl, Ethyl, n- oder i-Propyl, Benzyl oder -CH₂-O-C₁-C₄-Alkyl beziehungsweise -CH₂-O-C₆-C₁₀-Aryl.

Bei Substituenten in den β,γ-Stellungen kann es sich zum Beispiel um C₁-C₄-Alkyl, C₁-C₄-Alkoxy, Benzyloxy, oder -O-CH₂-O-, -O-CH(C₁-C₄-Alkyl)-O-, und -O-C(C₁-C₄-Alkyl)₂-O- handeln. Einige Beispiele sind Methyl, Ethyl, Methoxy, Ethoxy, -O-CH(Methyl)-O-, und -O-C(Methyl)₂-O-.

Je nach Art der Substitution und Anzahl der Substituenten können die zyklischen Phosphinreste C-chiral, P-chiral oder C- und P-chiral sein.

An zwei benachbarte C-Atome in den Resten der obigen Formeln kann ein aliphatischer 5-oder 6-Ring oder Benzol ankondensiert sein.

Das zyklische Sekundärphosphino kann zum Beispiel den Formeln (nur eines der möglichen Diastereomeren angegeben) entsprechen, 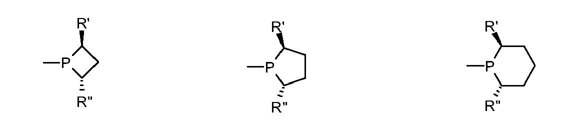 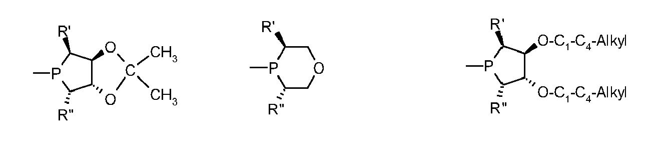 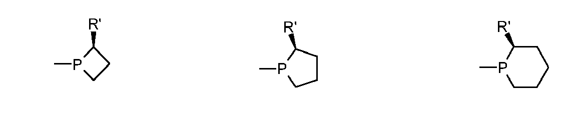 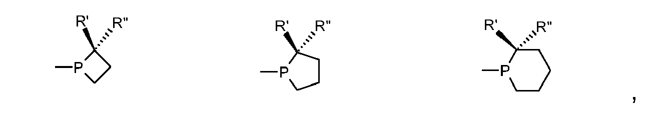 worin
die Reste R' und R" für C₁-C₄-Alkyl, zum Beispiel Methyl, Ethyl, n- oder i-Propyl, Benzyl, oder -CH₂-O-C₁-C₄-Alkyl beziehungsweise -CH₂-O-C₆-C₁₀-Aryl stehen, und R' und R" gleich oder voneinander verschieden sind.

In den Verbindungen der Formeln X bedeutet Phos als Sekundärphosphino bevorzugt nichtzyklisches Sekundärphosphin ausgewählt aus der Gruppe -P(C₁-C₆-Alkyl)₂, -P(C₅-C₈-Cycloalkyl)₂, -P(C₇-C₈-Bicycloalkyl)₂, -P(o-Furyl)₂, -P(C₆H₅)₂, -P[2-(C₁-C₆-Alkyl)C₆H₄]₂, -P[3-(C₁-C₆-Alkyl)C₆H₄]₂, -P[4-(C₁-C₆-Alkyl)C₆H₄]₂, -P[2-(Ci-C₆-Alkoxy)C₆H₄]₂, -P[3-(C₁-C₆-Alkoxy)C₆H₄]₂, -P[4-(C₁-C₆-Alkoxy)C₆H₄]₂, -P[2-(Trifluormethyl)C₆H₄]₂, -P[3-(Trifluormethyl)C₆H₄]₂, -P[4-(Trifluormethyl)C₆H₄]₂, -P[3,5-Bis(trifluormethyl)C₆H₃]₂, -P[3,5-Bis(C₁-C₆-Alkyl)₂C₆H₃]₂, -P[3,5-Bis(C₁-C₆-Alkoxy)₂C₆H₃]₂, und -P[3,5-Bis(C₁-C₆-Alkyl)₂-4-(C₁-C₆-Alkoxy)C₆H₂]₂, oder ein zyklisches Phosphin, ausgewählt aus der Gruppe 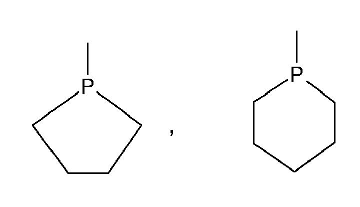 und 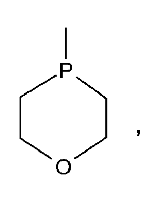 die unsubstituiert oder ein- oder mehrfach substituiert sind mit C₁-C₄-Alkyl, C₁-C₄-Alkoxy, C₁-C₄-Alkoxy-C₁-C₂-Alkyl, Phenyl, Benzyl, Benzyloxy, oder C₁-C₄-Alkyliden-dioxyl.

Einige spezifische Beispiele sind -P(CH₃)₂, -P(i-C₃H₇)₂, -P(n-C₄H₉)₂, -P(i-C₄H₉)₂, -P(t-C₄H₉)₂, -P(C₅H₉), -P(C₆H₁₁)₂, -P(Norbornyl)₂, -P(o-Furyl)₂, -P(C₆H₅)₂, P[2-(Methyl)C₆H₄]₂, P[3-(Methyl)C₆H₄]₂, -P[4-(Methyl)C₆H₄]₂, -P[2-(Methoxy)C₆H₄]₂, -P[3-(Methoxy)C₆H₄]₂, -P[4-(Methoxy)C₆H₄]₂, -P[3-(Trifluormethyl)C₆H₄]₂, -P[4-(Trifluormethyl)C₆H₄]₂, -P[3,5-Bis(trifluormethyl)-C₆H₃]₂, -P[3,5-Bis(methyl)₂C₆H₃]₂, -P[3,5-Bis(methoxy)₂C₆H₃]₂, und -P[3,5-Bis(methyl)₂-4-(methoxy)C₆H₂]₂, und solche der Formeln 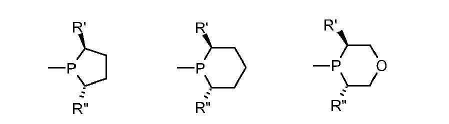 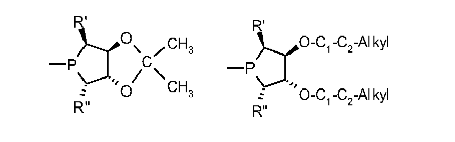 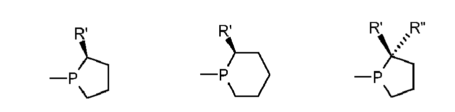 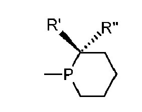 worin
R' Methyl, Ethyl, Methoxy, Ethoxy, Phenoxy, Benzyloxy, Methoxy-methyl, Ethoxymethyl oder Benzyloxymethyl darstellt und R" unabhängig die gleiche Bedeutung wie R' hat, aber von R' verschieden ist.

Die Verbindungen der Formel X können in einfacher und modularer Weise in hohen Ausbeuten selbst als entantiomerenreine Diastereomere hergestellt werden. Auch Zwischenprodukte sind schon als entantiomerenreine Diastereomere erhältlich, was die Herstellung von reinen diastereomeren Endprodukten erleichtert. Man geht zweckmässig von 1,1'-Dihalogenferrocen aus, das kommerziell erhältlich ist, zum Beispiel 1,1'-Dibromferrocen, und in dem man selektiv mit Metallierungsreagenzien wie zum Beispiel Li-Alkyl ein Halogen durch ein Metall substituiert werden kann.

In einer ersten Variante führt man dann durch Umsetzung mit R₂-P(Hal)₂ die Gruppe R₂HalP-ein. Die Umsetzung mit ortho-metallierten und mit Y substituierten Ferrocenen führt zu einem zentralen Zwischenprodukt der Formel VIII, das mittels Erwärmen und Umkristallisieren als reines Diastereomer erhältlich ist: 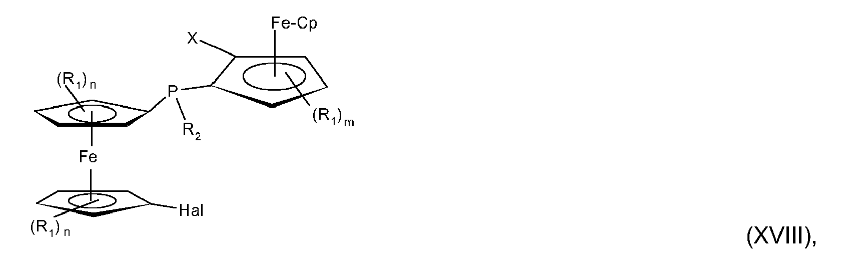 wobei Hal für Halogen (Cl, Br oder I, bevorzugt Br) steht. In den Verbindungen der Formel VIII können nach erneuter Metallierung (Substitution von Hal) mit Halogenphosphinen der Formel Phos-Hal eine gewünschte Gruppe Phos eingeführt werden.

In einer anderen Variante führt man durch Umsetzung mit Phos-Hal zuerst die Gruppe Phos ein, und dann mittels Metallierung und nachfolgender Umsetzung mit R₂-P(Hal)₂ die Gruppe R₂HalP-. Man erhält Zwischenprodukte der Formel XX, 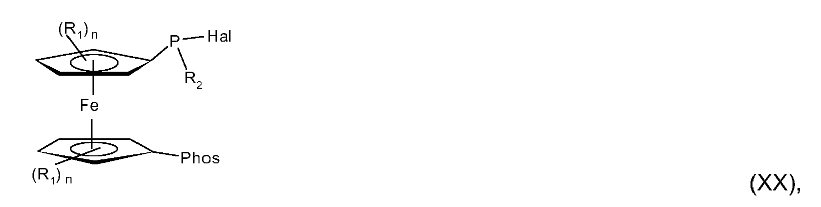 die in einer letzten mit ortho-metallierten und mit X substituierten Ferrocenen zu Verbindungen der Formel X umgesetzt werden. Die erhaltenen Gemische von Diastereomeren können durch Erwärmen und Umkristallisation in ein reines Diastereomer umgewandelt werden.

Ein Verfahren zur Herstellung von Verbindungen der Formel X umfasst, beispielsweise, die Schritte:
a) Metallierung eines 1,1'-Dihalogenferrocens zu einem 1-Metall-1'-halogenferrocen und nachfolgende Umsetzung, mit einer Verbindung der Formel R₂-P(Hal)₂, worin Halogen für Chlor, Brom oder lod steht, zu einer Verbindung der Formel XVI, 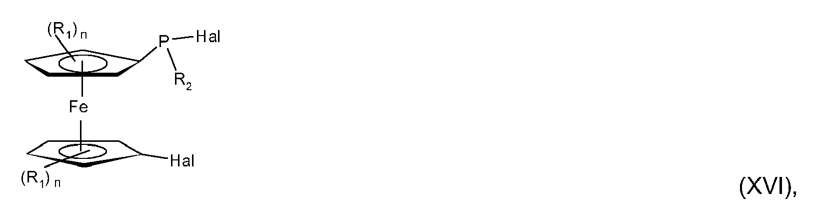 worin R₁, R₂ und n die zuvor angegebene Bedeutungen haben, und Hal für Chlor, Brom oder lod steht,
b) Umsetzung einer Verbindung der Formeln XVI mit einer Verbindung der Formel XVII 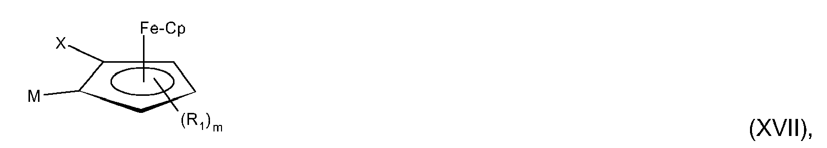 worin X, Cp, R₁ und m die zuvor angegebenen Bedeutungen haben und M für Li oder MgHal mit Hal gleich Chlor, Brom oder lod steht, zu einer Verbindung der Formel XVIII, 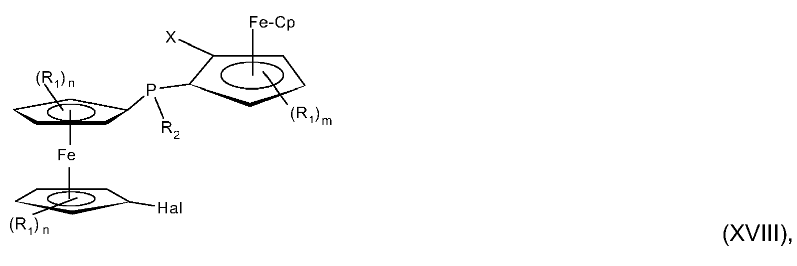 und
c) Umsetzung einer Verbindung der Formel VIII mit Lithiumalkyl und dann mit einem Halogenphosphin der Formel Phos-Hal, worin Hal für Chlor, Brom oder lod steht, zu einer Verbindung der Formel X.

Bei der Verfahrensstufe b) werden Gemische von Diastereomeren der P-chiralen Verbindungen der Formel XVIII erhalten. Gemische von Diastereomeren von Verbindungen der Formel XVIII gemäss Verfahrensstufe b) können mittels bekannter Methoden, zum Beispiel Chromatographie, in ihre verschiedenen Stereoisomeren getrennt werden.

Diese Gemische können aber auch in überraschend einfacher Weise durch blosse thermische Behandlung und gegebenenfalls einer nachfolgenden Umkristallisation in reine Diastereomere übergeführt werden. Eine thermische Behandlung und gegebenenfalls Umkristallisation empfiehlt sich vor Durchführung der Verfahrensstufe c) zur Vermeidung von Reiniungschritten wie Trennungen an chiralen Säulen nach Verfahrensstufe c) zur Herstellung reiner Diastereomerer. Thermische Behandlung kann zum Beispiel Aufnehmen des Reaktionsproduktes in einem inerten Lösungsmittel und ein Erwärmen auf 40 bis 150 °C und bevorzugt 60 bis 120 °C für einen Zeitraum von Minuten bis Stunden, zum Beispiel 10 Minuten bis 10 Stunden bedeuten. Geeignete Lösungsmittel sind später erwähnt.

Die in Verfahrensstufe a) verwendeten Dihalogenferrocene und Dihalogenphosphine sind bekannt, teilweise käuflich oder sie können nach analogen Verfahren hergestellt werden. Verbindungen der Formel XVIII sind bekannt oder nach bekannten beziehungsweise analogen Verfahren herstellbar. Man geht von bekannten X-substituierten Ferrocenen aus, die man in Orthostellung metalliert. Bei der Metallierung mit Lithiumalkyl oder auch Magnesium-Grignardverbindungen von Ferrocenen handelt es sich um bekannte Reaktionen, die zum Beispiel von T. Hayashi et al., Bull. Chem. Soc. Jpn. 53 (1980), Seiten 1138 bis 1151 oder in Jonathan Clayden Organolithiums: Selectivity for Synthesis (Tetrahedron Organic Chemistry Series), Pergamon Press (2002) beschrieben sind. Das Alkyl im Lithiumalkyl kann zum Beispiel 1 bis 6 C-Atome und bevorzugt 1 bis 4 C-Atome enthalten. Häufig wird Lithiummethyl, Lithium-s-butyl, Lithium-n-butyl und Lithium-t-butyl verwendet. Bei Magnesium-Grignardverbindungen handelt es sich bevorzugt um solche der Formel (C₁-C₄-Alkyl)MgX₀, worin X₀ Cl, Br oder I bedeutet.

Die Reaktionen in den Verfahrensstufen a), b) und c) werden zweckmässig bei niedrigen Temperaturen durchgeführt, zum Beispiel 20 bis -100 °C, bevorzugt 0 bis -80 °C. Nach Zugabe von Reaganzien kann die Temperatur auch erhöht werden, zum Beispiel auf Raumtemperatur. Die Reaktionen werden vorteilhaft unter inerten Schutzgasen durchgeführt, zum Beispiel Stickstoff oder Edelgasen wie zum Beispiel Helium oder Argon.

Bevorzugte Liganden entsprechen der Formel X, worin
m für 0 steht;
n für 0 steht;
R₂ C₁-C₆-Alkyl, C₅-C₈-Cycloalkyl, C₇-C₈-Bicycloalkyl, o-Furyl, Phenyl, Naphthyl, 2-(C₁-C₆-Alkyl)C₆H₄, 3-(C₁-C₆-Alkyl)C₆H₄, 4-(C₁-C₆-Alkyl)C₆H₄, 2-(C₁-C₆-Alkoxy)C₆H₄, 3-(C₁-C₆-Alkoxy)C₆H₄, 4-(C₁-C₆-Alkoxy)C₆H₄, 2-(Trifluormethyl)C₆H₄, 3-(Trifluormethyl)C₆H₄, 4-(Trifluormethyl)C₆H₄, 3,5-Bis(trifluormethyl)C₆H₃, 3,5-Bis(C₁-C₆-Alkyl)₂C₆H₃, 3,5-Bis(C₁-C₆-Alkoxy)₂C₆H₃ oder 3,5-Bis(C₁-C₆-Alkyl)₂-4-(C₁-C₆-Alkoxy)C₆H₂ bedeutet;
Cp für unsubstituiertes Cyclopentadienyl steht;
X eine Gruppe -CHR₅-NR₈R₉ darstellt, worin R₅ C₁-C₄-Alkyl, C₅-C₆-Cycloalkyl, Phenyl, C₁-C₄-Alkylphenyl oder C₁-C₄-Alkylbenzyl steht, und R₈ und R₉ gleich sind und C₁-C₄-Alkyl und bevorzugt Methyl oder Ethyl darstellen; und
Phos einen P-gebundenen P(III)-Substituenten der Formel -PR₃R₄ bedeutet, worin R₃ und R₄ unabhängig voneinander einen Rest, ausgewählt aus der Gruppe C₃-C₈-Alkyl, Cyclopentyl, Cyclohexyl, Furyl, Naphthyl, und unsubstituiertes oder mit ein bis drei F, Cl, C₁-C₄-Alkyl, C₁-C₄-Alkoxy und/oder C₁-C₄-Fluoralkyl substituiertes Phenyl.

Ganz besonders bevorzugt sind die Liganden der Formeln:

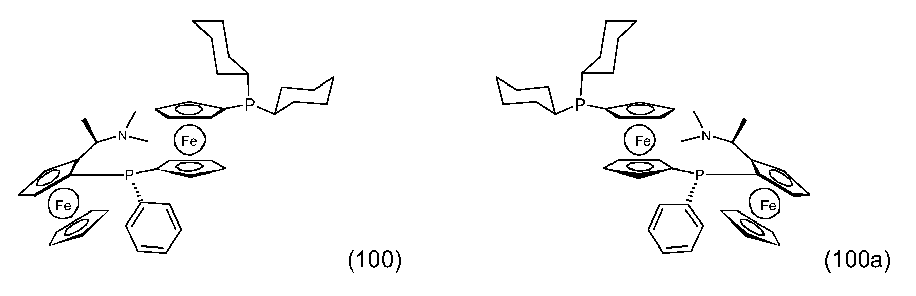

Asymmetrische Hydrierungen der Verfahrensstufe 2e) bzw. 3d) von α,β-ungesättigten Alkoholen der Formel VIII können allgemein bevorzugt unter Verwendung von Ruthenium-, Iridium- und Rhodiumkatalysatoren, wie zum Beispiel von M. Bänziger und T. Troxler in Tetrahedron: Asymmetry, Vol. 14 (2003) Seiten 3469-3477, R. Gilbertson in Tetrahedron Letters, Vol. 44 (2003) Seiten 953-955, P. G. Andersson im Journal of the American Chemical Society, Vol. 126 (2004), Seiten 14308-14309, A. Pfaltz im Organic Letters, Vol. 6 (2004), Seiten 2023-2026, und F. Spindler in Tetrahedron: Asymmetry, Vol. 15 (2004), Seiten 2299-2306, beschrieben, durchgeführt werden.

Liganden mit einem Ferrocenylgerüst sind im Allgemeinen für die asymmetrische Hydrierung von α,β-ungesättigten Alkoholen als Liganden für Rhodium geeignet und zum Beispiel von F. Spindler in Tetrahedron: Asymmetry, Vol. 15 (2004), Seiten 2299-2306, beschrieben. Beispiele sind Liganden der Klassen Walphos, Josiphos, Mandyphos und Taniaphos. Liganden dieser Klassen sind zum Beispiel von X. Zhang in Chemical Reviews, Vol. 103 (2003), Seiten 3029-3069 und von T. J. Colacot in Chemical Reviews, Vol. 103 (2003), Seiten 3101-3118, beschrieben.

Es wurde nun überraschend gefunden, dass Metallkomplexe der Formel XI mit Liganden vom Typ der Ferrocen-1,1'-diphosphine, die in 1-Stellung eine Ferrocen-substituierte, sekundäre Phosphingruppe und in 1 '-Stellung eine sekundäre Phosphingruppe aufweisen, besonders für die asymmetrische Hydrierung von α,β-ungesättigten Alkoholen geeignet sind. Gute optische Ausbeuten werden dabei mit Metallkomplexen der Formeln XI oder XIa erzielt,

[LM²X¹X²] (XI),

[LM²X¹]⁺E⁻ (XIa),

worin
M² Rhodium bedeutet;
X¹ für zwei Olefine oder ein Dien steht;
X² Cl, Br oder I bedeutet;
E⁻ das Anion einer Sauerstoffsäure oder Komplexsäure darstellt; und
L für einen chiralen Liganden aus der Gruppe der Ferrocen-1,1'-diphosphine, die in 1-Stellung eine Ferrocen-substituierte, sekundäre Phosphingruppe und in 1'-Stellung eine sekundäre Phosphingruppe aufweisen, steht.

Bei X¹ in der Bedeutung als Olefin kann es sich um C₂-C₁₂-, bevorzugt C₂-C₆- und besonders bevorzugt C₂-C₄-Olefine handeln. Beispiele sind Propen, Buten und besonders Ethylen. Das Dien kann 5 bis 12 und bevorzugt 5 bis 8 C-Atome enthalten und es kann sich um offenkettige, cyclische oder polycyclische Diene handeln. Die beiden Olefingruppen des Diens sind bevorzugt durch ein oder zwei CH₂-Gruppen verbunden. Beispiele sind 1,3-Pentadien, Cyclopentadien, 1,5-Hexadien, 1,4-Cyclohexadien, 1,4- oder 1,5-Heptadien, 1,4- oder 1,5-Cycloheptadien, 1,4- oder 1,5-Octadien, 1,4- oder 1,5-Cyclooctadien und Norbornadien. Bevorzugt stellt X¹ zwei Ethylen oder 1,5- Hexadien, 1,5-Cyclooctadien oder Norbornadien dar.

In Formel XI steht X² bevorzugt für Cl oder Br. Beispiele für E⁻ sind ClO₄⁻, CF₃SO₃⁻, CH₃SO₃⁻, HSO₄⁻, BF₄⁻, B(Phenyl)₄⁻, BARF (B(3,5-bis(Trifluormethyl)phenyl)₄⁻, PF₆⁻, SbCl₆⁻, AsF₆⁻ oder SbF₆⁻.

Mit den unten beschriebenen Liganden in den Metallkomplexen der Formeln XI und Xla können unerwartet hohe optische Ausbeuten erreicht werden, was für die Herstellung im industriellen Massstab einen erheblichen Vorteil (beispielsweise Kosteneinsparung) bedeutet. In den Verfahrensstufen 2e) bzw. 3d), ausgehend von α,β-ungesättigten Alkoholen der Formel VIII können daher zum Beispiel Metallkomplexe der Formeln XI und XIa eingesetzt werden, deren Liganden zu den Klassen der Ferrocen-1,1'-diphosphine, die in 1-Stellung eine Ferrocen-substituierte, sekundäre Phosphingruppe und in 1'-Stellung eine sekundäre Phosphingruppe aufweisen, gehören.

Im Rahmen der vorliegenden Erfindung sind Ferrocen-1,1'-diphosphine, die in 1-Stellung eine Ferrocen-substituierte, sekundäre Phosphingruppe und in 1'-Stellung eine sekundäre Phosphingruppeaufweisen, Verbindungen der Formel X in Form von enantiomerenreinen Diastereomeren oder einem Gemisch von Diastereomeren, 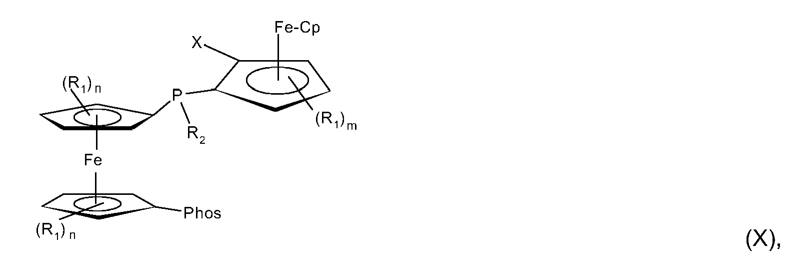 worin
die R₁ gleich oder verschieden sind und C₁-C₄-Alkyl bedeuten;
m für 0 steht oder eine ganze Zahl von 1 bis 3 darstellt;
n für 0 steht oder eine ganze Zahl von 1 bis 4 darstellt;
R₂ einen Kohlenwasserstoffrest oder C-gebundenen Heterokohlenwasserstoffrest bedeutet; Cp für unsubstituiertes oder mit C₁-C₄-Alkyl substituiertes Cyclopentadienyl steht;
X eine C-gebundene, Metalle von Metallierungsreagenzien in die Orthostellung dirigierende, chirale Gruppe darstellt; und
Phos einen P-gebundenen P(III)-Substituenten bedeutet.

Phos stellt als P-gebundener P(III)-Substituent besonders bevorzugt eine sekundäre Phosphingruppe dar.

Bei R₁ als Alkyl kann es sich zum Beispiel um Methyl, Ethyl, n- oder i-Propyl, n-, i- oder t-Butyl handeln, wobei Methyl bevorzugt ist. Bevorzugt stehen m und n für 0 (und R₁ ist damit ein Wasserstoffatom).

Die Kohlenwasserstoffreste R₂ können unsubstituiert oder substituiert sein und und/oder Heteroatome ausgewählt aus der Gruppe O, S, -N= oder N(C₁-C₄-Alkyl) enthalten. Sie können 1 bis 22, bevorzugt 1 bis 18, besonders bevorzugt 1 bis 12 und insbesondere bevorzugt 1 bis 8 C-Atome sowie 1 bis 4 und bevorzugt 1 oder 2 der genannten Heteroatome enthalten. Bei R₂ kann es sich um Reste, ausgewählt aus der Gruppe lineares oder verzweigtes C₁-C₁₂-Alkyl; unsubstituiertes oder mit C₁-C₆-Alkyl oder C₁-C₆-Alkoxy substituiertes C₄-C₁₂-Cycloalkyl oder C₄-C₁₂-Cycloalkyl-CH₂-; C₆-C₁₄-Aryl; C₄-C₁₂-Heteroaryl; C₇-C₁₄-Aralkyl; C₄-C₁₂-Heteroaralkyl; oder mit Halogen (Fluor, Chlor oder Brom), C₁-C₆-Alkyl, Trifluormethyl, C₁-C₆-Alkoxy, Trifluormethoxy, (C₆H₅)₃Si, (C₁-C₁₂-Alkyl)₃Si, oder Sekundäramino substituiertes C₆-C₁₄-Aryl, C₄-C₁₂-Heteroaryl, C₇-C₁₄-Aralkyl oder C₄-C₁₂-Heteroaralkyl handeln. Heteroaryl und Heteroaralkyl enthalten bevorzugt Heteroatome ausgewählt aus der Gruppe O, S und -N=.

Beispiele für R₂ als Alkyl, das bevorzugt 1 bis 6 C-Atome enthält, sind Methyl, Ethyl, n-Propyl, i-Propyl, n-Butyl, i-Butyl, t-Butyl, und die Isomeren von Pentyl und Hexyl. Beispiele für R₂ als gegebenenfalls mit Alkyl substituiertes Cycloalkyl sind Cyclopentyl, Cyclohexyl, Methyl-und Ethylcyclohexyl, Dimethylcyclohexyl, Cycloheptyl, Cyclooctyl, Norbornyl und Adamantyl. Beispiele für R₂ als gegebenenfalls mit Alkyl, und Alkoxy substituiertes C₅-C₁₂-Cycloalkyl-CH₂- sind Cyclopentylmethyl, Cyclohexylmethyl, Cyclooctylmethyl, Methylcyclohexylmethyl und Dimethylcyclohexylmethyl. Beispiele für R₂ als Aryl und Aralkyl sind Phenyl, Naphthyl, Anthracenyl, Fluorenyl, Benzyl und Naphthylmethyl. Beispiele für R₂ als Heteroaryl und Heteroaralkyl sind Furyl, Thiophenyl, N-Methylpyrrolidinyl, Pyridyl, Benzofuranyl, Benzothiophenyl, Chinonlinyl, Furylmethyl, Thiophenylmethyl und Pyridylmethyl. Beispiele für R₂ als substituiertes Aryl, Aralkyl, Heteroaryl und Heteroaralkyl sind Phenyl, Naphthyl, Benzyl, Naphthylmethyl, Phenylethyl, Furyl, Thiophenyl, Benzofuryl und Benzothiophenyl, die mit 1 bis 3 Resten ausgewählt aus der Gruppe Methyl, Ethyl, n- und i-Propyl, n-, i- und t-Butyl, Ethoxy, Methoxy, Trifluormethyl, Trifluormethoxy, Fluor oder Chlor substituiert sind. Einige bevorzugte Beispiele sind 2-, 3- oder 4-Methylphenyl, 2,4- oder 3,5-Dimethylphenyl, 3,4,5-Trimethylphenyl, 4-Ethylphenyl, 2- oder 4-Methylbenzyl, 2-, 3- oder 4-Methoxyphenyl, 2,4- oder 3,5-Dimethoxyphenyl, 3,4,5-Trimethoxyphenyl, 2-, 3- oder 4-Trifluormethylphenyl, 2,4- oder 3,5-Di(trifluormethyl)phenyl, Tris-trifluormethylphenyl, 2- oder 4-Trifluormethoxyphenyl, 3,5-Bis-trifluormethoxyphenyl, 2- oder 4-Fluor-phenyl, 2- oder 4-Chlorphenyl und 3,5-Dimethyl-4-methoxyphenyl.

In einer besonders bevorzugten Ausführungsform bedeutet R₂ C₁-C₆-Alkyl, C₅-C₈-Cycloalkyl, C₇-C₈-Bicycloalkyl, o-Furyl, Phenyl, Naphthyl, 2-(C₁-C₆-Alkyl)C₆H₄, 3-(C₁-C₆-Alkyl)C₆H₄, 4-(C₁-C₆-Alkyl)C₆H₄, 2-(C₁-C₆-Alkoxy)C₆H₄, 3-(C₁-C₆-Alkoxy)C₆H₄, 4-(C₁-C₆-Alkoxy)C₆H₄, 2-(Trifluormethyl)C₆H₄, 3-(Trifluormethyl)C₆H₄, 4-(Trifluormethyl)C₆H₄, 3,5-Bis(trifluormethyl)C₆H₃, -3,5-Bis(C₁-C₆-Alkyl)₂C₆H₃, 3,5-Bis(C₁-C₆-Alkoxy)₂C₆H₃, und 3,5-Bis(C₁-C₆-Alkyl)₂-4-(C₁-C₆-Alkoxy)C₆H₂.

Bei der orthodirigierenden, chiralen Gruppe X ist das chirale Atom vorzugsweise in 1-, 2-oder 3-Stellung zur Bindung Cyclopentadienyl-X gebunden. Bei der Gruppe X kann es sich um offenkettige Reste oder zyklische Reste handeln, wobei die Atome ausgewählt sind aus der Gruppe H, C, O, S und N.

Die Gruppe X kann zum Beispiel der Formel -HC*R₅R₆ (mit * wird das asymmetrische Atom gekennzeichnet) entsprechen, worin R₅ C₁-C₈-Alkyl, C₅-C₈-Cycloalkyl (Cyclohexyl), C₆-C₁₀-Aryl (Phenyl), C₇-C₁₂-Aralkyl (Benzyl) oder C₇-C₁₂-Alkaralkyl (Methylbenzyl) steht, R₆ für -OR₇ oder-NR₈R₉ steht, R₇ C₁-C₈-Alkyl, C₅-C₈-Cycloalkyl, Phenyl oder Benzyl bedeutet, und R₈ und R₉ gleich oder verschieden sind und C₁-C₈-Alkyl, C₅-C₈-Cycloalkyl, Phenyl oder Benzyl darstellen oder R₈ und R₉ zusammen mit dem N-Atom einen fünf- bis achtgliedrigen Ring bilden. R₅ ist bevorzugt C₁-C₄-Alkyl wie zum Beispiel Methyl, Ethyl, n-Propyl und Phenyl. R₇ bedeutet bevorzugt C₁-C₄-Alkyl wie zum Beispiel Methyl, Ethyl, n-Propyl und n- oder i-Butyl. R₈ und R₉ bedeuten bevorzugt gleiche Reste und stellen bevorzugt C₁-C₄-Alkyl wie zum Beispiel Methyl, Ethyl, n-Propyl, i-Propyl und n- oder i-Butyl und zusammen Teramethylen, Pentamethylen oder 3-Oxa-1,5-pentylen dar. Besonders bevorzugte Gruppen der Formel -HCR₅R₆ sind 1-Methoxy-eth-1-yl, 1-Dimethylamino-eth-1-yl und 1-(Dimethylamino)-1-phenyl-methyl.

X stellt besonders bevorzugt eine Gruppe -CHR₅-NR₈R₉ dar, worin R₅ C₁-C₄-Alkyl, C₅-C₆-Cycloalkyl, Phenyl, C₁-C₄-Alkylphenyl oder C₁-C₄-Alkylbenzyl steht, und R₈ und R₉ gleich sind und C₁-C₄-Alkyl und bevorzugt Methyl oder Ethyl darstellen.

Phos kann als P-gebundener P(III)-Substituent eine sekundäre Phosphingruppe sein, die gleiche oder verschiedene Kohlenwasserstoffreste enthält, oder in denen der Kohlenwasserstoffrest zusammen mit dem P-Atom einen 4- bis 8-gliedrigen Ring bildet. Bevorzugt enthält die sekundäre Phosphingruppe gleiche Kohlenwasserstoffreste. Die Kohlenwasserstoffreste können unsubstituiert oder substituiert sein und und/oder Heteroatome ausgewählt aus der Gruppe O, S, -N= oder N(C₁-C₄-Alkyl) enthalten. Sie können 1 bis 22, bevorzugt 1 bis 18, besonders bevorzugt 1 bis 12 und insbesondere bevorzugt 1 bis 8 C-Atome 1 bis 4 und bevorzugt 1 oder 2 der genannten Heteroatome enthalten. Es kann sich um Reste, ausgewählt aus der Gruppe lineares oder verzweigtes C₁-C₁₂-Alkyl; unsubstituiertes oder mit C₁-C₆-Alkyl oder C₁-C₆-Alkoxy substituiertes C₅-C₁₂-Cycloalkyl oder C₅-C₁₂-Cycloalkyl-CH₂-; C₆-C₁₄-Aryl; C₄-C₁₂-Heteroaryl; C₇-C₁₄-Aralkyl; C₄-C₁₂-Heteroaralkyl; oder mit Halogen (Fluor, Chlor oder Brom), C₁-C₆-Alkyl, Trifluormethyl, C₁-C₆-Alkoxy, Trifluormethoxy, (C₆H₅)₃Si, (C₁-C₁₂-Alkyl)₃Si, oder Sekundäramino substituiertes C₆-C₁₄-Aryl, C₄-C₁₂-Heteroaryl, C₇-C₁₄-Aralkyl oder C₄-C₁₂-Heteroaralkyl handeln. Heteroaryl und Heteroaralkyl enthalten bevorzugt Heteroatome ausgewählt aus der Gruppe O, S und -N=.

Beispiele für Kohlenwasserstoffreste als Alkyl, das bevorzugt 1 bis 6 C-Atome enthält, sind Methyl, Ethyl, n-Propyl, i-Propyl, n-Butyl, i-Butyl, t-Butyl, und die Isomeren von Pentyl und Hexyl. Beispiele für Kohlenwasserstoffreste als gegebenenfalls mit Alkyl substituiertes Cycloalkyl sind Cyclopentyl, Cyclohexyl, Methyl- und Ethylcyclohexyl, Dimethylcyclohexyl, Cycloheptyl, Cyclooctyl, Norbornyl und Adamantyl. Beispiele für Kohlenwasserstoffreste als gegebenenfalls mit Alkyl, und Alkoxy substituiertes C₅-C₁₂-Cycloalkyl-CH₂- sind Cyclopentylmethyl, Cyclohexylmethyl, Cyclooctylmethyl, Methylcyclohexylmethyl und Dimethylcyclohexylmethyl. Beispiele für Kohlenwasserstoffreste als Aryl und Aralkyl sind Phenyl, Naphthyl, Anthracenyl, Fluorenyl, Benzyl und Naphthylmethyl. Beispiele für Kohlenwasserstoffreste als Heteroaryl und Heteroaralkyl sind Furyl, Thiophenyl, N-Methylpyrrolidinyl, Pyridyl, Benzofuranyl, Benzothiophenyl, Chinonlinyl, Furylmethyl, Thiophenylmethyl und Pyridylmethyl. Beispiele für Kohlenwasserstoffreste als substituiertes Aryl, Aralkyl, Heteroaryl und Heteroaralkyl sind Phenyl, Naphthyl, Benzyl, Naphthylmethyl, Phenylethyl, Furyl, Thiophenyl, Benzofuryl und Benzothiophenyl, die mit 1 bis 3 Resten ausgewählt aus der Gruppe Methyl, Ethyl, n- und i-Propyl, n-, i- und t-Butyl, Ethoxy, Methoxy, Trifluormethyl, Trifluormethoxy, Fluor oder Chlor substituiert sind. Einige bevorzugte Beispiele sind 2-, 3- oder 4-Methylphenyl, 2,4-oder 3,5-Dimethylphenyl, 3,4,5-Trimethylphenyl, 4-Ethylphenyl, 2- oder 4-Methylbenzyl, 2-, 3- oder 4-Methoxyphenyl, 2,4- oder 3,5-Dimethoxyphenyl, 3,4,5-Trimethoxyphenyl, 2-, 3-oder 4-Trifluormethylphenyl, 2,4- oder 3,5-Di(trifluormethyl)phenyl, Tris-trifluormethylphenyl, 2- oder 4-Trifluormethoxyphenyl, 3,5-Bis-trifluormethoxyphenyl, 2- oder 4-Fluor-phenyl, 2-oder 4-Chlorphenyl und 3,5-Dimethyl-4-methoxyphenyl.

In einer besonders bevorzugten Ausführungsform bedeutet der Kohlenwasserstoffrest C₁-C₆-Alkyl, C₅-C₈-Cycloalkyl, C₇-C₈-Bicycloalkyl, o-Furyl, Phenyl, Naphthyl, 2-(C₁-C₆-Alkyl)C₆H₄, 3-(C₁-C₆-Alkyl)C₆H₄, 4-(C₁-C₆-Alkyl)C₆H₄, 2-(C₁-C₆-Alkoxy)C₆H₄, 3-(C₁-C₆-Alkoxy)C₆H₄, 4-(C₁-C₆-Alkoxy)C₆H₄, 2-(Trifluormethyl)C₆H₄, 3-(Trifluormethyl)C₆H₄, 4-(Trifluormethyl)C₆H₄, 3,5-Bis(trifluormethyl)C₆H₃, -3,5-Bis(C₁-C₆-Alkyl)₂C₆H₃, 3,5-Bis(C₁-C₆-Alkoxy)₂C₆H₃, und 3,5-Bis(C₁-C₆-Alkyl)₂-4-(C₁-C₆-Alkoxy)C₆H₂.

Die Sekundärphosphinogruppe entspricht bevorzugt der Formel -PR₃R₄, worin R₃ und R₄ unabhängig voneinander einen Kohlenwasserstoffrest mit 1 bis 18 C-Atomen darstellen, der unsubstituiert oder substituiert ist mit C₁-C₆-Alkyl, Trifluormethyl, C₁-C₆-Alkoxy, Trifluormethoxy, (C₁-C₄-Alkyl)₂amino, (C₆H₅)₃Si, (C₁-C₁₂-Alkyl)₃Si, Halogen, und/oder Heteroatome O enthält.

Bevorzugt sind R₃ und R₄ Reste, ausgewählt aus der Gruppe lineares oder verzweigtes C₁-C₆-Alkyl, unsubstituiertes oder mit ein bis drei C₁-C₄-Alkyl oder C₁-C₄-Alkoxy substituiertes Cyclopentyl oder Cyclohexyl, Furyl, unsubstituiertes oder mit ein bis drei C₁-C₄-Alkyl oder C₁-C₄-Alkoxy substituiertes Benzyl, und insbesondere unsubstituiertes oder mit ein bis drei F, Cl, C₁-C₄-Alkyl, C₁-C₄-Alkoxy, C₁-C₄-Fluoralkyl oder C₁-C₄-Fluoralkoxy substituiertes Phenyl oder Naphthyl.

Besonders bevorzugt bedeuten R₃ und R₄ Reste, ausgewählt aus der Gruppe C₃-C₈-Alkyl, Cyclopentyl, Cyclohexyl, Furyl, Naphthyl, und unsubstituiertes oder mit ein bis drei F, Cl, C₁-C₄-Alkyl, C₁-C₄-Alkoxy und/oder C₁-C₄-Fluoralkyl substituiertes Phenyl.

Bei der sekundären Phosphingruppe Phos kann es sich um zyklisches Sekundärphosphino handeln, zum Beispiel solche der Formeln 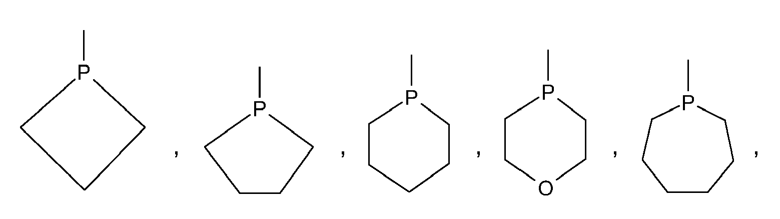 die unsubstituiert oder ein- oder mehrfach substituiert sind mit C₁-C₈-Alkyl, C₄-C₈-Cycloalkyl, C₁-C₆-Alkoxy, C₁-C₄-Alkoxy-C₁-C₄-Alkyl, Phenyl, C₁-C₄-Alkyl- oder C₁-C₄-Alkoxyphenyl, Benzyl, C₁-C₄-Alkyl- oder C₁-C₄-Alkoxybenzyl, Benzyloxy, C₁-C₄-Alkyl- oder C₁-C₄-Alkoxybenzyloxy, oder C₁-C₄-Alkyliden-dioxyl.

Die Substituenten können in einer oder beiden α-Stellungen zum P-Atom gebunden sein, um chirale C-Atome einzuführen. Bei den Substituenten in einer oder beiden α-Stellungen handelt es sich bevorzugt um C₁-C₄-Alkyl oder Benzyl, zum Beispiel um Methyl, Ethyl, n- oder i-Propyl, Benzyl oder -CH₂O-C₁-C₄-Alkyl beziehungsweise -CH₂-C₆-C₁₀-Aryl.

Bei Substituenten in den β,γ-Stellungen kann es sich zum Beispiel um C₁-C₄-Alkyl, C₁-C₄-Alkoxy, Benzyloxy, oder -O-CH₂-O-, -O-CH(C₁-C₄-Alkyl)-O-, und -O-C(C₁-C₄-Alkyl)₂-O- handeln. Einige Beispiele sind Methyl, Ethyl, Methoxy, Ethoxy, -O-CH(Methyl)-O-, und -O-C(Methyl)₂-O-.

Je nach Art der Substitution und Anzahl der Substituenten können die zyklischen Phosphinreste C-chiral, P-chiral oder C- und P-chiral sein.

An zwei benachbarte C-Atome in den Resten der obigen Formeln kann ein aliphatischer 5-oder 6-Ring oder Benzol ankondensiert sein.

Das zyklische Sekundärphosphino kann zum Beispiel den Formeln (nur eines der möglichen Diastereomeren angegeben) entsprechen, 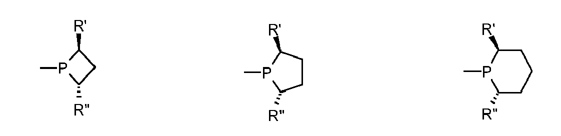 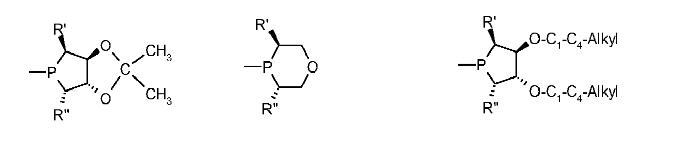 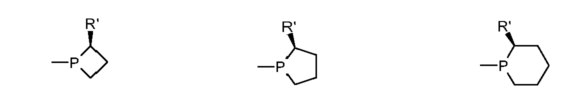 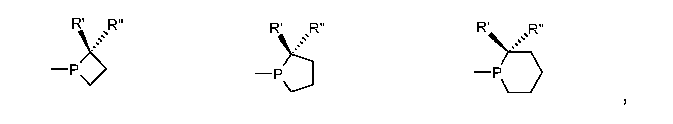 worin
die Reste R' und R" für C₁-C₄-Alkyl, zum Beispiel Methyl, Ethyl, n- oder i-Propyl, Benzyl, oder -CH₂-O-C₁-C₄-Alkyl beziehungsweise -CH₂-O-C₆-C₁₀-Aryl stehen, und R' und R" gleich oder voneinander verschieden sind.

In den Verbindungen der Formeln X bedeutet Phos als Sekundärphosphino bevorzugt nichtzyklisches Sekundärphosphin ausgewählt aus der Gruppe -P(C₁-C₆-Alkyl)₂, -P(C₅-C₈-Cycloalkyl)₂, -P(C₇-C₈-Bicydoalkyl)₂, -P(o-Furyl)₂, -P(C₆H₅)₂, -P[2-(C₁-C₆-Alkyl)C₆H₄]₂, -P[3-(C₁-C₆-Alkyl)C₆H₄]₂, -P[4-(C₁-C₆-Alkyl)C₆H₄]₂, -P[2-(C₁-C₆-Alkoxy)C₆H₄]₂, -P[3-(C₁-C₆-Alkoxy)C₆H₄]₂, -P[4-(C₁-C₆-Alkoxy)C₆H₄]₂, -P[2-(Trifluormethyl)C₆H₄]₂, -P[3-(Trifluormethyl)C₆H₄]₂, -P[4-(Trifluormethyl)C₆H₄]₂, -P[3,5-Bis(trifluormethyl)C₆H₃]₂, -P[3,5-Bis(C₁-C₆-Alkyl)₂C₆H₃]₂, -P[3,5-Bis(C₁-C₆-Alkoxy)₂C₆H₃]₂, und -P[3,5-Bis(C₁-C₆-Alkyl)₂-4-(C₁-C₆-Alkoxy)C₆H₂]₂, oder ein zyklisches Phosphin, ausgewählt aus der Gruppe 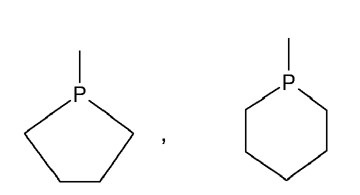 und 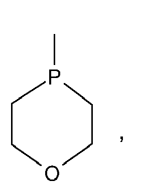 die unsubstituiert oder ein- oder mehrfach substituiert sind mit C₁-C₄-Alkyl, C₁-C₄-Alkoxy, C₁-C₄-Alkoxy-C₁-C₂-Alkyl, Phenyl, Benzyl, Benzyloxy, oder C₁-C₄-Alkyliden-dioxyl.

Einige spezifische Beispiele sind -P(CH₃)₂, -P(i-C₃H₇)₂, -P(n-C₄H₉)₂, -P(i-C₄H₉)₂, -P(t-C₄H₉)₂, -P(C₅H₉), -P(C₆H₁₁)₂, -P(Norbornyl)₂, -P(o-Furyl)₂, -P(C₆H₅)₂, P[2-(Methyl)C₆H₄]₂, P[3-(Methyl)C₆H₄]₂, -P[4-(Methyl)C₆H₄]₂, -P[2-(Methoxy)C₆H₄]₂, -P[3-(Methoxy)C₆H₄]₂, -P[4-(Methoxy)C₆H₄]₂, -P[3-(Trifluormethyl)C₆H₄]₂, -P[4-(Trifluormethyl)C₆H₄]₂, -P[3,5-Bis(trifluormethyl)-C₆H₃]₂, -P[3,5-Bis(methyl)₂C₆H₃]₂, -P[3,5-Bis(methoxy)₂C₆H₃]₂, und -P[3,5-Bis(methyl)₂-4-(methoxy)C₆H₂]₂, und solche der Formeln 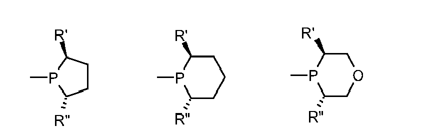 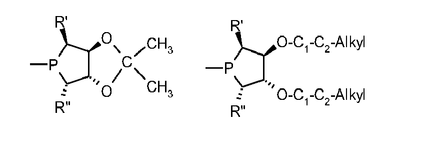 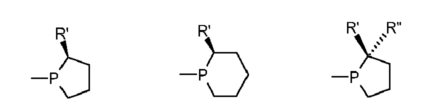 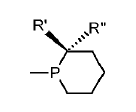 worin
R' Methyl, Ethyl, Methoxy, Ethoxy, Phenoxy, Benzyloxy, Methoxy-methyl, Ethoxymethyl oder Benzyloxymethyl darstellt und R" unabhängig die gleiche Bedeutung wie R' hat, aber von R' verschieden ist.

Die Verbindungen der Formel X können in einfacher und modularer Weise in hohen Ausbeuten selbst als entantiomerenreine Diastereomere hergestellt werden. Auch Zwischenprodukte sind schon als entantiomerenreine Diastereomere erhältlich, was die Herstellung von reinen diastereomeren Endprodukten erleichtert. Man geht zweckmässig von 1,1'-Dihalogenferrocen aus, das kommerziell erhältlich ist, zum Beispiel 1,1'-Dibromferrocen, und in dem man selektiv mit Metallierungsreagenzien wie zum Beispiel Li-Alkyl ein Halogen durch ein Metall substituiert werden kann.

In einer ersten Variante führt man dann durch Umsetzung mit R₂-P(Hal)₂ die Gruppe R₂HalP-ein. Die Umsetzung mit ortho-metallierten und mit Y substituierten Ferrocenen führt zu einem zentralen Zwischenprodukt der Formel VIII, das mittels Erwärmen und Umkristallisieren als reines Diastereomer erhältlich ist: 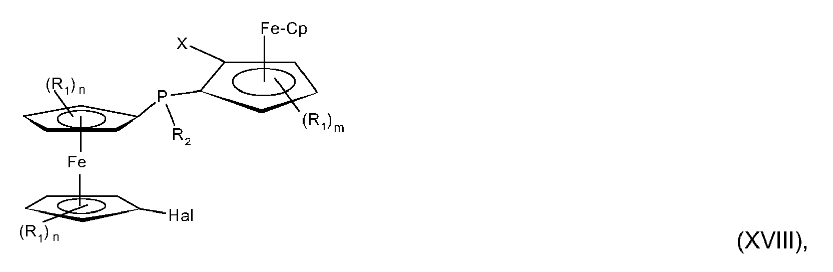 wobei Hal für Halogen (Cl, Br oder I, bevorzugt Br) steht. In den Verbindungen der Formel VIII können nach erneuter Metallierung (Substitution von Hal) mit Halogenphosphinen der Formel Phos-Hal eine gewünschte Gruppe Phos eingeführt werden.

In einer anderen Variante führt man durch Umsetzung mit Phos-Hal zuerst die Gruppe Phos ein, und dann mittels Metallierung und nachfolgender Umsetzung mit R₂-P(Hal)₂ die Gruppe R₂HalP-. Man erhält Zwischenprodukte der Formel XX, 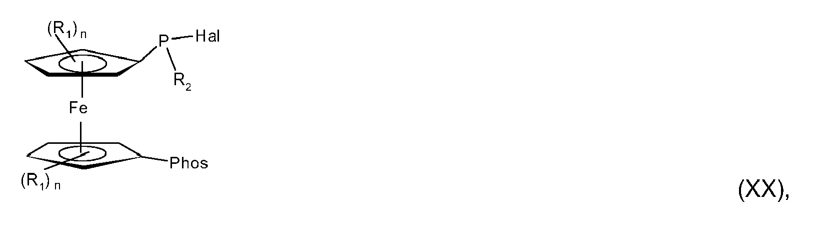 die in einer letzten mit ortho-metallierten und mit X substituierten Ferrocenen zu Verbindungen der Formel X umgesetzt werden. Die erhaltenen Gemische von Diastereomeren können durch Erwärmen und Umkristallisation in ein reines Diastereomer umgewandelt werden.

Ein Verfahren zur Herstellung von Verbindungen der Formel X umfasst, beispielsweise, die Schritte:
a) Metallierung eines 1,1'-Dihalogenferrocens zu einem 1-Metall-1'-halogenferrocen und nachfolgende Umsetzung, mit einer Verbindung der Formel R₂-P(Hal)₂, worin Halogen für Chlor, Brom oder lod steht, zu einer Verbindung der Formel XVI, 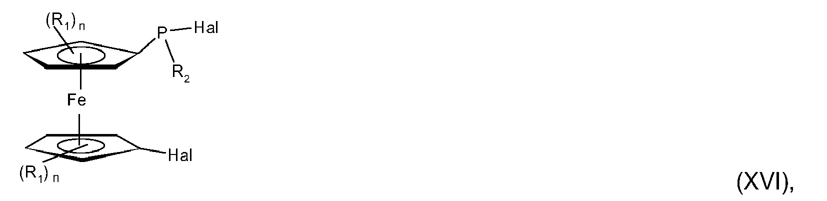 worin R₁, R₂ und n die zuvor angegebene Bedeutungen haben, und Hal für Chlor, Brom oder lod steht,
b) Umsetzung einer Verbindung der Formeln XVI mit einer Verbindung der Formel XVII 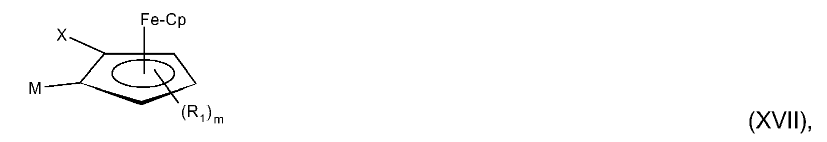 worin X, Cp, R₁ und m die zuvor angegebenen Bedeutungen haben und M für Li oder MgHal mit Hal gleich Chlor, Brom oder Iod steht, zu einer Verbindung der Formel XVIII, 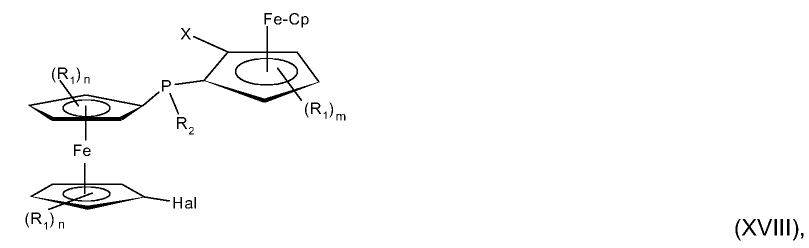 und
c) Umsetzung einer Verbindung der Formel VIII mit Lithiumalkyl und dann mit einem Halogenphosphin der Formel Phos-Hal, worin Hal für Chlor, Brom oder lod steht, zu einer Verbindung der Formel X.

Bei der Verfahrensstufe b) werden Gemische von Diastereomeren der P-chiralen Verbindungen der Formel XVIII erhalten. Gemische von Diastereomeren von Verbindungen der Formel XVIII gemäss Verfahrensstufe b) können mittels bekannter Methoden, zum Beispiel Chromatographie, in ihre verschiedenen Stereoisomeren getrennt werden.

Diese Gemische können aber auch in überraschend einfacher Weise durch blosse thermische Behandlung und gegebenenfalls einer nachfolgenden Umkristallisation in reine Diastereomere übergeführt werden. Eine thermische Behandlung und gegebenenfalls Umkristallisation empfiehlt sich vor Durchführung der Verfahrensstufe c) zur Vermeidung von Reiniungschritten wie Trennungen an chiralen Säulen nach Verfahrensstufe c) zur Herstellung reiner Diastereomerer. Thermische Behandlung kann zum Beispiel Aufnehmen des Reaktionsproduktes in einem inerten Lösungsmittel und ein Erwärmen auf 40 bis 150 °C und bevorzugt 60 bis 120 °C für einen Zeitraum von Minuten bis Stunden, zum Beispiel 10 Minuten bis 10 Stunden bedeuten. Geeignete Lösungsmittel sind später erwähnt.

Die in Verfahrensstufe a) verwendeten Dihalogenferrocene und Dihalogenphosphine sind bekannt, teilweise käuflich oder sie können nach analogen Verfahren hergestellt werden. Verbindungen der Formel XVIII sind bekannt oder nach bekannten beziehungsweise analogen Verfahren herstellbar. Man geht von bekannten X-substituierten Ferrocenen aus, die man in Orthostellung metalliert. Bei der Metallierung mit Lithiumalkyl oder auch Magnesium-Grignardverbindungen von Ferrocenen handelt es sich um bekannte Reaktionen, die zum Beispiel von T. Hayashi et al., Bull. Chem. Soc. Jpn. 53 (1980), Seiten 1138 bis 1151 oder in Jonathan Clayden Organolithiums: Selectivity for Synthesis (Tetrahedron Organic Chemistry Series), Pergamon Press (2002) beschrieben sind. Das Alkyl im Lithiumalkyl kann zum Beispiel 1 bis 6 C-Atome und bevorzugt 1 bis 4 C-Atome enthalten. Häufig wird Lithiummethyl, Lithium-s-butyl, Lithium-n-butyl und Lithium-t-butyl verwendet. Bei Magnesium-Grignardverbindungen handelt es sich bevorzugt um solche der Formel (C₁-C₄-Alkyl)MgX₀, worin X₀ Cl, Br oder I bedeutet.

Die Reaktionen in den Verfahrensstufen a), b) und c) werden zweckmässig bei niedrigen Temperaturen durchgeführt, zum Beispiel 20 bis -100 °C, bevorzugt 0 bis -80 °C. Nach Zugabe von Reaganzien kann die Temperatur auch erhöht werden, zum Beispiel auf Raumtemperatur. Die Reaktionen werden vorteilhaft unter inerten Schutzgasen durchgeführt, zum Beispiel Stickstoff oder Edelgasen wie zum Beispiel Helium oder Argon.

Bevorzugte Liganden entsprechen der Formel X, worin
m für 0 steht;
n für 0 steht;
R₂ C₁-C₆-Alkyl, C₅-C₈-Cycloalkyl, C₇-C₈-Bicycloalkyl, o-Furyl, Phenyl, Naphthyl, 2-(C₁-C₆-Alkyl)C₆H₄, 3-(C₁-C₆-Alkyl)C₆H₄, 4-(C₁-C₆-Alkyl)C₆H₄, 2-(C₁-C₆-Alkoxy)C₆H₄, 3-(C₁-C₆-Alkoxy)C₆H₄, 4-(C₁-C₆-Alkoxy)C₆H₄, 2-(Trifluormethyl)C₆H₄, 3-(Trifluormethyl)C₆H₄, 4-(Trifluormethyl)C₆H₄, 3,5-Bis(trifluormethyl)C₆H₃, 3,5-Bis(C₁-C₆-Alkyl)₂C₆H₃, 3,5-Bis(C₁-C₆-Alkoxy)₂C₆H₃ oder 3,5-Bis(C₁-C₆-Alkyl)₂-4-(C₁-C₆-Alkoxy)C₆H₂ bedeutet;
Cp für unsubstituiertes Cyclopentadienyl steht;
X eine Gruppe -CHR₅-NR₈R₉ darstellt, worin R₅ C₁-C₄-Alkyl, C₅-C₆-Cycloalkyl, Phenyl, C₁-C₄-Alkylphenyl oder C₁-C₄-Alkylbenzyl steht, und R₈ und R₉ gleich sind und C₁-C₄-Alkyl und bevorzugt Methyl oder Ethyl darstellen; und
Phos einen P-gebundenen P(III)-Substituenten der Formel -PR₃R₄ bedeutet, worin R₃ und R₄ unabhängig voneinander einen Rest, ausgewählt aus der Gruppe C₃-C₈-Alkyl, Cyclopentyl, Cyclohexyl, Furyl, Naphthyl, und unsubstituiertes oder mit ein bis drei F, Cl, C₁-C₄-Alkyl, C₁-C₄-Alkoxy und/oder C₁-C₄-Fluoralkyl substituiertes Phenyl.

Ganz besonders bevorzugt sind die Liganden der Formeln

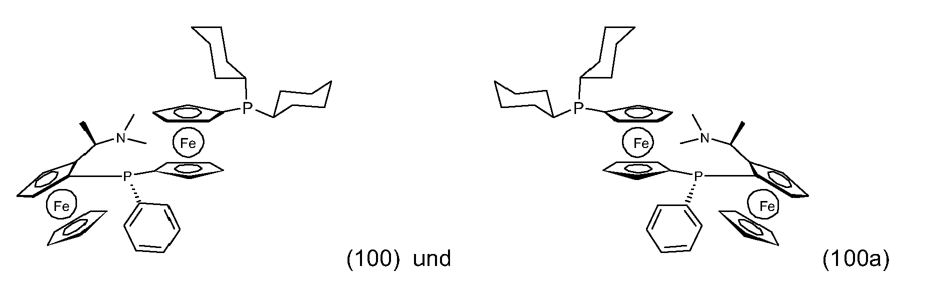

Die in den Verfahrensstufen 1d), 2e) bzw. 3d) als Katalysatoren verwendeten Metallkomplexe können als getrennt hergestellte isolierte Verbindungen zugegeben werden, oder auch in situ vor der Reaktion gebildet und dann mit dem zu hydrierenden Substrat vermischt werden. Es kann vorteilhaft sein, bei der Reaktion unter Verwendung von isolierten Metallkomplexen zusätzlich Liganden zuzugeben, oder bei der in situ Herstellung einen Überschuss der Liganden einzusetzen. Der Überschuss kann zum Beispiel bis 10 Mol und vorzugsweise 0,001 bis 5 Mol betragen, bezogen auf die zur Herstellung verwendete Metallverbindung.

Die Verfahrensstufen 1d), 2e) bzw. 3d) können bei Normaldruck oder bevorzugt bei Überdruck durchgeführt werden. Der Druck kann zum Beispiel von 10⁵ bis 2x10⁷ Pa (Pascal) betragen.

Katalysatoren, die zur Hydrierung in den Verfahrensstufen 1d), 2e) bzw. 3d) eingesetzt werden, werden bevorzugt in Mengen von 0,0001 bis 10 Mol-%, besonders bevorzugt 0,001 bis 10 Mol-%, und insbesondere bevorzugt 0,01 bis 5 Mol-% verwendet, bezogen auf die zu hydrierende Verbindung.

Die Herstellung der Katalysatoren sowie die Verfahrensstufen 1d), 2e) bzw. 3d) und die anderen Verfahrensstufen können ohne oder in Gegenwart eines inerten Lösungsmittels durchgeführt werden, wobei ein Lösungsmittel oder Gemische von Lösungsmitteln eingesetzt werden können. Geeignete Lösungsmittel sind zum Beispiel aliphatische, cycloaliphatiche und aromatische Kohlenwasserstoffe (Pentan, Hexan, Petrolether, Cyclohexan, Methylcyclohexan, Benzol, Toluol, Xylol), aliphatische Halogenkohlenwasserstoffe (Methylenchlorid, Chloroform, Di- und Tetrachlorethan), Nitrile (Acetonitril, Propionitril, Benzonitril), Ether (Diethylether, Dibutyl-ether, t-Butylmethylether, Ethylenglykoldimethylether, Ethylenglykoldiethylether, Diethylen-glykoldimethylether, Tetrahydrofuran, Dioxan, Diethylenglykolmonomethyl- oder monoethyl-ether), Ketone (Aceton, Methylisobutylketon), Carbonsäureester und Lactone (Essigsäure-ethyl- oder -methylester, Valerolacton), N-substituierte Lactame (N-Methylpyrrolidon), Car-bonsäureamide (Dimethylamid, Dimethylformamid), acyclische Harnstoffe (Dimethylimi-dazolin), und Sulfoxide und Sulfone (Dimethylsulfoxid, Dimethylsulfon, Tetramethylensulf-oxid, Tetramethylensulfon) und Alkohole (Methanol, Ethanol, Propanol, Butanol, Ethylen-glykolmonomethylether, Ethylenglykolmonoethylether, Diethylenglykolmonomethylether) und Wasser. Die Lösungsmittel können alleine oder in Mischung von wenigstens zwei Lösungsmitteln verwendet werden.

Die Reaktion der Verfahrensstufen 1d), 2e) bzw. 3d) kann in Gegenwart von Co-Katalysatoren durchgeführt werden, zum Beispiel quaternären Ammoniumhalogeniden (Tetrabutylammoniumiodid) und/oder in Gegenwart von Protonensäuren, zum Beispiel Mineralsäuren, durchgeführt werden.

Die Verfahrensstufen 1e) und 2d) werden vorzugsweise bei niedrigen Temperaturen, zum Beispiel -40°C bis 0°C, und vorteilhaft in einem Lösungsmittel durchgeführt. Geeignete Lösungsmittel sind zum Beispiel Ether (Tetrahydrofuran oder Dioxan). Zur Reduktion werden zweckmässig Metallhydride in wenigstens äquimolaren Mengen verwendet, zum Beispiel BH₃•S(CH₃)₂, LiAlH₄, NaBH₄ + TiCl₄, NaBH₄ + AlCl₃, NaBH₄ + BF₃•Et₂O, LiAlH(OMe)₃, AlH₃, sowie Alkylmetallhydride wie Diisobutyl-aluminiumhydrid.

Die Verfahrensstufe 3c) wird vorzugsweise bei niedrigen Temperaturen, zum Beispiel -40°C bis 0°C, und vorteilhaft in einem Lösungsmittel durchgeführt. Geeignete Lösungsmittel sind zum Beispiel Kohlenwasserstoffe (Pentan, Cyclohexan, Methylcyclohexan, Benzol, Toluol und Xylol). Zur Reduktion werden zweckmässig Metallhydride in wenigstens äquimolaren Mengen verwendet, zum Beispiel, NaBH₄, LiAlH₄, AlH₃, sowie Alkylmetallhydride wie beispielsweise Diisobutylaluminium-hydrid und Tributylzinnhydrid.

Mit dem erfindungsgemässen regiospezifischen, bzw. regioselektiven und enantioselektiven Verfahren können die Zwischenprodukte zur Herstellung der Verbindung der Formel (B) über alle Verfahrensstufen in hohen Ausbeuten hergestellt werden. Die hohen Gesamtausbeuten machen das Verfahren für den industriellen Einsatz geeignet.

Die nachfolgenden Beispiele erläutern die Erfindung näher.

HPLC-Gradient auf Hypersil BDS C-18 (5 µm); Säule: 4 x 125 mm
(I) 90% Wasser*/10% Acetonitril* zu 0% Wasser*/100% Acetonitril* in 5 Minuten + 2.5 Minuten (1.5 ml/min)
(II) 95% Wasser*/5% Acetonitril* zu 0% Wasser*/100% Acetonitril* in 40 Minuten (0.8 ml/min)
HPLC-Gradient auf Synergi Polar-RP 80A(Phenomenex) (4 um); Säule: 4.60 x 100 mm
(III) 90% Wasser*/10% Acetonitril* zu 0% Wasser*/100% Acetonitril* in 5 Minuten + 2.5 Minuten (1.5 ml/min)
(IV) 95% Wasser*/5% Acetonitril* zu 0% Wasser*/100% Acetonitril* in 40 Minuten (0.8 ml/min)
* enthält 0.1 % Trifluoressigsäure

### Beispiel A)

Verfahren zur Herstellung von (R)-2-[1-(3-Methoxy-propyl)-3-methyl-1 H-indazol-6-ylmethyl]-3-methyl-butan-1-ol (A6)

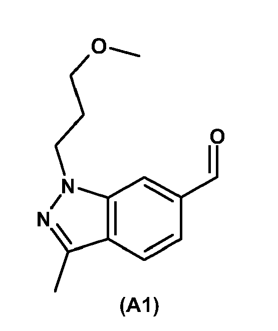

### Beispiel A1:

### Herstellung von 1-(3-Methoxy-propyl)-3-methyl-1 H-indazol-6-carbaldehyd

Eine Lösung von 10.0 g 6-Brom-1-(3-methoxy-propyl)-3-methyl-1 H-indazol (WO 2005/090305 A1) in 70 ml Tetrahydrofuran wird auf -78°C gekühlt und mit 3.76 ml N-Methylmorpholin versetzt. Es wird erneut auf -78°C gekühlt und 23 ml n-Butyllithium (1,6 M in Hexan) so zugetropft, dass die Innentemperatur -70°C nicht übersteigt. Es wird bei -70°C während 2 Minuten nachgerührt. Anschliessend werden 5.18 ml N,N-Dimethylformamid so zugetropft, dass die Innentemperatur -70°C nicht übersteigt. Es wird bei -70°C während 5 Minuten nachgerührt. Zur Reaktionsmischung werden 70 ml 1M wässrige Ammoniumchlorid-Lösung zugegeben und die Reaktionsmischung wird auf Raumtemperatur erwärmt. Das Reaktionsgemisch wird mit 50 ml Wasser verdünnt und anschliessend mit tert-Butyl methylether (2 x 100 ml) extrahiert. Die organischen Phasen werden mit Sole (1 x 100 ml) gewaschen. Die vereinigten organischen Phasen werden mit Natriumsulfat getrocknet, filtriert und am Rotationsverdampfer eingedampft. Aus dem Rückstand wird die rohe Titelverbindung A1 als gelbes Oel erhalten. Gehalt (NMR): 90% (enthält 10% 1-(3-Methoxy-propyl)-3-methyl-1 H-indazol). (7.80 g, 90.4 %). Rf = 0.27 (Essigester-Heptan 1:1); Rt = 3.67 (Gradient I).

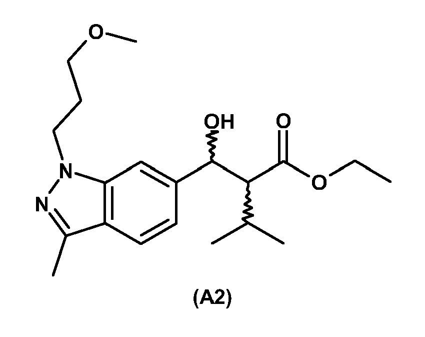

### Beispiel A2:

### Herstellung von 2-{Hydroxy-[1-(3-methoxy-propyl)-3-methyl-1H-indazol-6-yl]-methyl}-3-methyl-buttersäure ethylester

Eine Lösung von 2.628 ml Diisopropylamin und 20 ml Tetrahydrofuran wird auf -20°C gekühlt und während 7 Minuten 11.508 ml n-Butyllithium (1,6 M in Hexan) zugetropft. Es wird bei -20°C 10 Minuten nachgerührt. Anschliessend wird eine Lösung von 2.62 ml Isovaleriansäureethylester in 15 ml Tetrahydrofuran während 10 Minuten bei -20°C zugetropft. Nach weiteren 5 Minuten wird eine Lösung von 3.60 g 1-(3-Methoxy-propyl)-3-methyl-1 H-indazol-6-carbaldehyd (A1) in 15 ml Tetrahydrofuran zugetropft und während 30 Minuten bei -20°C nachgerührt. Dann werden 30 ml gesättigte, wässrige Ammoniumchlorid-Lösung zugetropft und anschliessend mit tert-Butyl methylether (2 x 100 ml) extrahiert. Die organischen Phasen werden nacheinander mit 0,5 N Salzsäure (1 x 100 ml) und Sole (1 x 50 ml) gewaschen. Die vereinigten organischen Phasen werden mit Natriumsulfat getrocknet, filtriert und am Rotationsverdampfer eingedampft. Aus dem Rückstand wird die rohe Titelverbindung A2 als weisser Feststoff erhalten (4.98 g, 89.6 %, syn:anti = 67:33). Rf = 0.08 (Essigester-Heptan 1:2); Rt = 15.96, 16.75 (Gradient II).

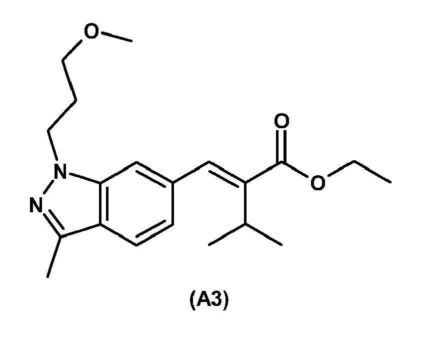

### Beispiel A3:

### Herstellung von 2-[1-[1-(3-Methoxy-propyl)-3-methyl-1 H-indazol-6-yl]-meth-(E)-yliden]-3-methyl-buttersäure ethylester

Eine Lösung von 2.80 g 2-{Hydroxy-[1-(3-methoxy-propyl)-3-methyl-1H-indazol-6-yl]-methyl}-3-methyl-buttersäure ethylester (A2) und 47 mg 4-Dimethylaminopyridin in 20 ml Tetrahydrofuran wird auf 0°C gekühlt. Man tropft 0.79 ml Essigsäureanhydrid über 2 Minuten zu und rührt das Reaktionsgemisch während 1 Stunde bei 0°C. Dann wird eine Lösung von 2.63 g Kalium-tert-butylat in 20 ml Tetrahydrofuran während 1 Stunde bei 0°C zugetropft und anschliessend 1 Stunde bei 0°C gerührt. Das Reaktionsgemisch wird auf 100 ml Eiswasser gegossen und mit tert-Butyl methylether (2 x 80 ml) extrahiert. Die organischen Phasen werden nacheinander mit 80 ml Wasser und 80 ml Sole gewaschen, mit Natriumsulfat getrocknet, filtriert und am Rotationsverdampfer eingedampft. Aus dem Rückstand wird mittels Flashchromatographie (SiO₂ 60F, Essigester-Hexan 1:6) die reine Titelverbindung A3 als leicht gelbliches Oel erhalten (1.83 g, 70%). Rf = 0.28 (Essigester-Heptan 1:2); Rt = 22.42 (Gradient II).

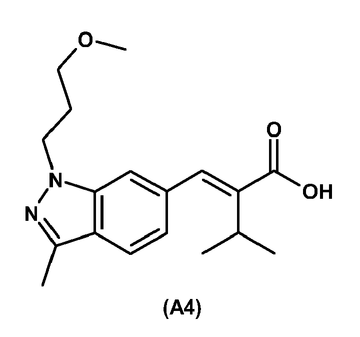

### Beispiel A4:

### Herstellung von 2-[1-[1-(3-Methoxy-propyl)-3-methyl-1 H-indazol-6-yl]-meth-(E)-yliden]-3-methyl-buttersäure

Eine Lösung von 2.80 g 2-{Hydroxy-[1-(3-methoxy-propyl)-3-methyl-1 H-indazol-6-yl]-methyl}-3-methyl-buttersäure ethylester (A2) und 47 mg 4-Dimethylaminopyridin in 20 ml Tetrahydrofuran wird auf 0°C gekühlt. Man tropft 0.79 ml Essigsäureanhydrid über 2 Minuten zu und rührt das Reaktionsgemisch während 1 Stunde bei 0°C. Dann wird eine Lösung von 2.63 g Kalium-tert-butylat in 20 ml Tetrahydrofuran während 1 Stunde bei 0°C zugetropft und anschliessend 1 Stunde bei 0°C gerührt. Man tropft 10 ml Eiswasser über 1 Minute zum Reaktionsgemisch und dampft am Rotationsverdampfer das Tetrahydrofuran ab. Die wässrige Emulsion wird mit 28 ml Ethanol und 3.8 ml 2M wässriger Kaliumhydroxid-Lösung versetzt und für 13.5 Stunden zum Rückfluss erhitzt. Aus dem Reaktionsgemisch wird am Rotationsverdampfer (35°C) das Ethanol eingedampft. Die resultierende wässrige Lösung wird mit tert-Butyl methylether (2 x 15 ml) gewaschen. Die wässrige Phase wird mit 10 ml 2M wässriger Salzsäure-Lösung angesäuert und mit tert-Butyl methylether (2 x 30 ml) extrahiert. Die organischen Phasen werden nacheinander mit 15 ml Wasser und 15 ml Sole gewaschen, mit Natriumsulfat getrocknet, filtriert und am Rotationsverdampfer eingedampft. Aus dem Rückstand wird mittels Kristallisation aus heissem Essigester-Heptan Gemisch die reine Titelverbindung A4 als weisse Kristalle erhalten (1.44 g, 60.1 %). Rf = 0.27 (Essigester-Heptan 3:1); Rt = 16.41 (Gradient II).

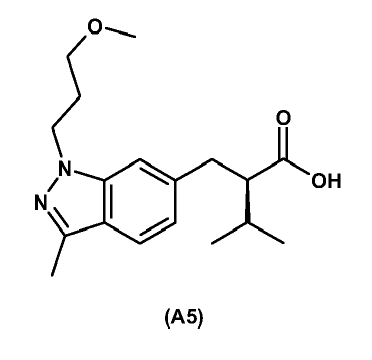

### Beispiel A5:

### (R)-2-[1-(3-Methoxy-propyl)-3-methyl-1 H-indazol-6-ylmethyl]-3-methyl-buttersäure

Die Titelverbindung ist durch katalytische asymmetrische Hydrierung von 2-[1-[1-(3-Methoxy-propyl)-3-methyl-1 H-indazol-6-yl]-meth-(E)-yliden]-3-methyl-buttersäure (A4) erhältlich.

Die asymetrischen Hydrierungen von 2-[1-[1-(3-Methoxy-propyl)-3-methyl-1 H-indazol-6-yl]-meth-(E)-yliden]-3-methyl-buttersäure (A4) werden in einer voll automatisierten high throughput screening Anlage, die von der Firma Symyx entwickelt wurde ausgeführt.

Das Reaktionsgemisch wird mittels der unten genannten HPLC Methode auf Umsatz und Enantiomeren-Überschuss hin untersucht. Dazu werden 80 µl der Reaktionslösung in 1000 µl Ethanol gelöst. Folgende Ergebnisse werden erzielt:

| **Ligand** | **Metall-Presursor** | **Verhältnis Substrat/Katalysator** | **Lösungsmittel** | **Zusatz** | **Umsatz** | **ee% (Absolute Konfiguration)** |
|---|---|---|---|---|---|---|
| 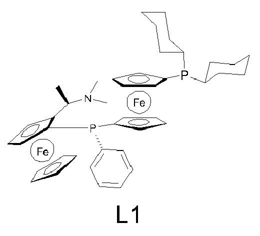 | [Rh(NBD)Cl₂] | 25 | MeOH | | >95 | 97.9 (R)* |
| | [Rh(NBD)₂]BF₄ | 25 | MeOH | | >95 | 96.1 (R)* |
| | [Rh(NBD)₂]BF₄ | 100 | MeOH | | >95 | 96.0 (R)* |
| | [Rh(NBD)₂]BF₄ | 200 | MeOH | | >95 | 96.4 (R)* |
| | [Rh(NBD)(OOCF₃)]₂ | 25 | MeOH | | >95 | 96.3 (R)* |
| | [Rh(NBD)Cl]₂ | 25 | EtOH | | >95 | 95.2 (R)* |
| | [lr(COD)₂]BARF | 25 | THF | | >95 | 96.3 (R)* |

| | | | | | | |
|---|---|---|---|---|---|---|
| Bedingungen: 41.66 µmol 2-[1-[1-(3-Methoxy-propyl)-3-methyl-1 H-indazol-6-yl]-meth-(E)-yliden]-3-methyl-buttersäure (A4); 500 µl Lösungsmittel; 1.2 Equivalente Ligand pro Metall; p(H₂): 20 bar; T: Raumtemperatur; Reaktionszeit: 16 Stunden * Unter ansonsten identischen Bedingungen wird mit dem enantiomeren Liganden das Produkt mit der (S)-Konfiguration erhalten. | | | | | | |

### HPLC Bedingungen:

| | |
|---|---|
| Instrument | SFC Berger Instruments |
| Säule | CHIRALPAK-AD (250 mm * 0.46 cm) |
| Modifier | Ethanol |
| Outlet pressure | 100 bar |
| Gradient | 15% EtOH 8', 60%/min 40%, 2' 40%, 60%/min 15%, 15% 6', |
| | Total 17' |
| Fluss | 1.5 ml/min. |
| Detektion | UV (210 nm) |
| Temperatur | 40°C |
| Probenkonzentration | 2 mg Produkt in 1.0 ml MeOH |
| Injektionsvolumen | 5.0 µL Loop |
| Laufzeit | 12 min. |

Retentionszeiten:
- (S)-**(A5)** 4.7 min
- (R)-**(A5)** 5.8 min
- **(A4)** 8.5 min

### Repräsentative Beschreibung der Reaktionsdurchführung in grösserem Massstab:

In einem 50 ml Autoklaven aus rostfreiem Edelstahl werden 3.161 mmol 2-[1-[1-(3-Methoxy-propyl)-3-methyl-1H-indazol-6-yl]-meth-(E)-yliden]-3-methyl-buttersäure (A4) vorgelegt. In einem 20 ml Schlenk-Rohr unter Argon Atmosphäre wird eine Lösung von 0.016 mmol des Liganden (L1) und 0.08 mmol des Metall-Precursors ([Rh(NBD)Cl]₂) in 15 ml entgastem, trockenem Methanol hergestellt und die Mischung 1.5 Stunden bei Raumtemperatur gerührt. Die Katalysatorlösung wird eine Kanüle in den 50 ml Autoklaven transferiert, welcher zuvor unter Argon Atmosphäre gesetzt wurde. Der Autoklav wird verschlossen und mit Argon gespült (es wird 3 mal jeweils ein Druck von 10 - 12 Bar angelegt und wieder auf 1 Bar entspannt). Anschliessend wird das Argon durch Wasserstoff ersetzt und es wird mit Wasserstoff gespült (es wird 3 mal jeweils ein Druck von 20 Bar angelegt und wieder auf 1 Bar entspannt). Der Autoklav wird anschliessend mit Wasserstoff unter einen Druck von 20 Bar gesetzt. Nach 19 Stunden wird der Druck abgelassen. Die Reaktionslösung wird am Rotationsverdampfer eingedampft. Reinigung des Rückstands mittels Flashchromatographie (SiO₂ 60F, Essigester) in Form von weissen Kristallen erhältlich (Ausbeute 2.93 mmol).
Rf = 0.32 (Essigester-Heptan 2:1); Rt = 4.03 (Gradient I).

Das Reaktionsgemisch wird mittels der oben genannten HPLC Methode auf Umsatz und Enantiomeren-Überschuss hin untersucht. Dazu werden 80 µl der Reaktionslösung in 1000 µl Ethanol gelöst. Folgende Ergebnisse werden erzielt:
Umsatz: 100%
ee% (Absolute Konfiguration: 98.9 (R)

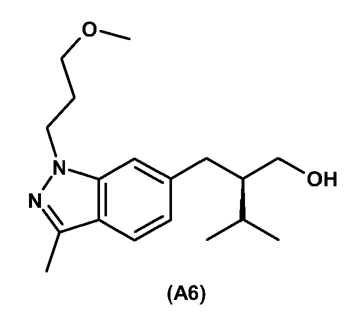

### Beispiel A6:

### (R)-2-[1-(3-Methoxy-propyl)-3-methyl-1 H-indazol-6-ylmethyl]-3-methyl-butan-1-ol

### a) Herstellung ausgehend von (R)-2-[1-(3-Methoxy-propyl)-3-methyl-1 H-indazol-6-ylmethyl]-3-methyl-buttersäure (A5)

Eine Lösung von 8.55 g (R)-2-[1-(3-Methoxy-propyl)-3-methyl-1 H-indazol-6-ylmethyl]-3-methyl-buttersäure (A5) in 85 ml Tetrahydrofuran wird auf 0°C gekühlt und mit 81.4 ml Boran-Tetrahydrofuran Komplex (1 M in Tetrahydrofuran) versetzt. Das Reaktionsgemisch wird während 17 Stunden bei Raumtemperatur gerührt. Die Reaktionsmischung wird auf 0°C abgekühlt und anschliessend langsam mit 100 ml Methanol versetzt. Das Gemisch wird am Rotationsverdampfer eingedampft und am Hochvakuum getrocknet. Aus dem Rückstand wird mittels Flashchromatographie (SiO₂ 60F, Essigester-Hexan 2:1) die reine Titelverbindung A6 als farbloses Oel erhalten (8.05 g, 98%). Rf = 0.28 (Essigester-Heptan 2:1); Rt = 4.13 (Gradient I).

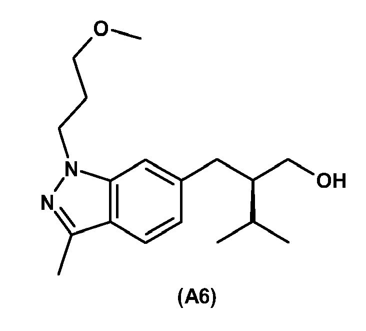

### Beispiel A6:

### (R)-2-[1-(3-Methoxy-propyl)-3-methyl-1 H-indazol-6-ylmethyl]-3-methyl-butan-1-ol

### b) Herstellung ausgehend von 2-[1-[1-(3-Methoxy-propyl)-3-methyl-1 H-indazol-6-yl]-meth-(E)-yliden]-3-methyl-butan-1-ol (A7)

Die Titelverbindung ist durch katalytische asymmetrische Hydrierung von 2-[1-[1-(3-Methoxy-propyl)-3-methyl-1 H-indazol-6-yl]-meth-(E)-yliden]-3-methyl-butan-1-ol (A7) und Reinigung der Rückstands mittels Flashchromatographie (SiO₂ 60F, Essigester) als leicht rosa Oel erhältlich. Rf = 0.32 (Essigester-Heptan 2:1); Rt = 4.13 (Gradient I).

Die asymetrischen Hydrierungen von 2-[1-[1-(3-Methoxy-propyl)-3-methyl-1 H-indazol-6-yl]-meth-(E)-yliden]-3-methyl-butan-1-ol (A7) werden in einer voll automatisierten high throughput screening Anlage, die von der Firma Symyx entwickelt wurde, ausgeführt.

Bedingungen: 41.66 umol Substrat, 500 µl Lösungsmittel, 1.2 Equivalente Ligand pro Metall. Das Reaktionsgemisch wird mittels der unten genannten HPLC Methode auf Umsatz und Enantiomeren-Überschuss hin untersucht. Dazu werden 80 µl der Reaktionslösung in 1000 µl Ethanol gelöst. Folgende Ergebnisse werden erzielt:

| **Ligand** | **Metall-Presursor** | **Verhältnis Substrat/ Katalysator** | **Lösungsmittel** | **Zusatz** | **Umsatz** | **ee% (Absolute Konfiguration)** |
|---|---|---|---|---|---|---|
| 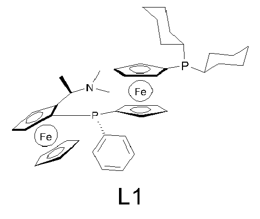 | [Rh(NBD)₂]BF₄ | 25 | THF | | 100 | 98.0 (R)* |
| | [Rh(NBD)₂]BF₄ | 25 | EtOH | | 100 | 97.8 (R)* |
| | [Rh(NBD)₂]BF₄ | 200 | THF | | 100 | 98.2 (R)* |

| | | | | | | |
|---|---|---|---|---|---|---|
| Bedingungen: 41.66 µmol 2-[1-[1-(3-Methoxy-propyl)-3-methyl-1H-indazol-6-yl]-meth-(E)-yliden]-3-methyl-butan-1-ol (A7); 500 µL Lösungsmittel; 1.2 Equivalente Ligand pro Metall; p(H₂): 80 bar; T: 40°C; Reaktionszeit: 14 Stunden * Unter ansonsten identischen Bedingungen wird mit dem enantiomeren Liganden das Produkt mit der (S)-Konfiguration erhalten. | | | | | | |

### HPLC Bedingungen:

| | |
|---|---|
| Instrument | SFC Berger Instruments |
| Säule | CHIRALPAK-AD (250 mm * 0.46 cm) |
| Modifier | Ethanol |
| Outlet pressure | 100 bar |
| Gradient | 15% EtOH 8', 60%/min 40%, 2' 40%, 60%/min 15%, 15% 6', |
| | Total 17' |
| Fluss | 1.5 ml/min. |
| Detektion | UV (210 nm) |
| Temperatur | 40°C |
| Probenkonzentration | 2 mg Produkt in 1.0 ml MeOH |
| Injektionsvolumen | 5.0 µL Loop |
| Laufzeit | 12 min. |

Retentionszeiten:
- (S)-**(A6)** 6.1 min
- (R)**-(A6)** 7.5 min
- **(A7)** 8.2 min

### Repräsentative Beschreibung der Reaktionsdurchführung in grösserem Massstab:

In einem Schlenk-Rohr wird eine Lösung aus 1.65 mmol 2-[1-[1-(3-Methoxy-propyl)-3-methyl-1H-indazol-6-yl]-meth-(E)-yliden]-3-methyl-butan-1-ol (A7) in 4 ml entgastem, trockenem Ethanol hergestellt und 10 Minuten bei Raumtemperatur gerührt. In einem zweiten Schlenk-Rohr unter Argon Atmosphäre wird eine Lösung der passenden Menge des Liganden (L1) und des Metall-Precursors ([Rh(NBD)₂]BF₄) 1.05 Equivalente Ligand pro Metall) in 4 ml entgastem, trockenem Ethanol hergestellt und die Mischung 10 Minuten bei Raumtemperatur gerührt. Beide Lösungen werden über eine Kanüle in einen 50 ml Autoklaven aus rostfreiem Edelstahl transferiert, welcher zuvor unter Argon Atmosphäre gesetzt wurde. Der Autoklav wird verschlossen und mit Argon gespült (es wird 4 mal jeweils ein Druck von 10 - 12 Bar angelegt und wieder auf 1 Bar entspannt). Anschliessend wird das Argon durch Wasserstoff ersetzt und es wird mit Wasserstoff gespült (es wird 4 mal jeweils ein Druck von 10 - 12 Bar angelegt und wieder auf 1 Bar entspannt). Der Autoklav wird anschliessend mit Wasserstoff unter einen Druck von 80 Bar gesetzt und auf 40°C erwärmt. Nach 20 Stunden wird auf Raumtemperatur abgekühlt und der Druck abgelassen. Die Reaktionslösung wird am Rotationsverdampfer eingedampft. Reinigung des Rückstands mittels Flashchromatographie (SiO₂ 60F, Essigester) als leicht rosa Oel erhältlich (Ausbeute 1.29 mmol). Rf = 0.32 (Essigester-Heptan 2:1); Rt = 4.13 (Gradient I).

Das Reaktionsgemisch wird mittels der oben genannten HPLC Methode auf Umsatz und Enantiomeren-Überschuss hin untersucht. Dazu werden 80 µl der Reaktionslösung in 1000 µl Ethanol gelöst. Folgende Ergebnisse werden erzielt:
Umsatz: 100%
ee% (Absolute Konfiguration: 97.8 (R)

Alternativ kann die Titelverbindung (A6) durch Reduktion von 2-[1-[1-(3-Methoxy-propyl)-3-methyl-1H-indazol-6-yl]-meth-(E)-yliden]-3-methyl-buttersäure (A4) zu 2-[1-[1-(3-Methoxy-propyl)-3-methyl-1H-indazol-6-yl]-meth-(E)-yliden]-3-methyl-butan-1-ol (A7), und nachfolgender katalytischer, asymmetrischer Hydrierung erhalten werden.

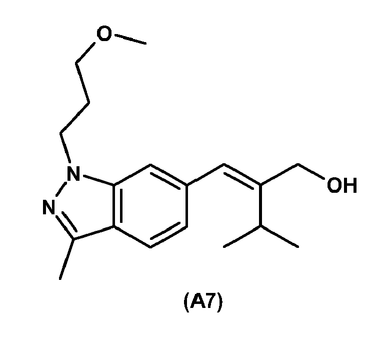

### Beispiel A7:

### 2-[1-[1-(3-Methoxy-propyl)-3-methyl-1 H-indazol-6-yl]-meth-(E)-yliden]-3-methyl-butan-1-ol

### a) Herstellung ausgehend von 2-[1-[1-(3-Methoxy-propyl)-3-methyl-1 H-indazol-6-yl]-meth-(E)-yliden]-3-methyl-buttersäure (A4)

Eine Lösung von 470 mg (2-[1-[1-(3-Methoxy-propyl)-3-methyl-1H-indazol-6-yl]-meth-(E)-yliden]-3-methyl-buttersäure (A4) in 2 ml Tetrahydrofuran wird auf 0°C gekühlt und mit einer Lösung von 56.4 mg Lithiumaluminiumhydrid in 2.5 ml Tetrahydrofuran versetzt. Das Reaktionsgemisch wird während 19 Stunden bei Raumtemperatur gerührt. Es werden anschliessend 50.0 mg festes Lithiumaluminiumhydrid bei Raumtemperatur zugegeben und das Reaktionsgemisch wird während 2 Stunden bei Raumtemperatur gerührt. Die Reaktionsmischung wird langsam mit 3 ml Eisessig versetzt. Das Gemisch wird mit Natriu-Katium Tartrat Lösung gewaschen, die wässrigen Phasen werden mit tert Butylmethylether extrahiert und die vereinigten organischen Phasen werden mit Natriumsulfat getrocknet, filtriert und am Rotationsverdampfer eingedampft und am Hochvakuum getrocknet. Aus dem Rückstand wird mittels Flashchromatographie (SiO₂ 60F, Essigester-Hexan 2:1) die reine Titelverbindung A7 als gelber Feststoff erhalten (252.7 mg, 56%). Rf = 0.29 (Essigester-Heptan 2:1); Rt = 3.96 (Gradient I).

Alternativ kann die Titelverbindung (A6) durch katalytische Reduktion von 2-[1-[1-(3-Methoxy-propyl)-3-methyl-1H-indazol-6-yl]-meth-(E)-yliden]-3-methyl-buttersäure ethylester (A3) zu 2-[1-[1-(3-Methoxy-propyl)-3-methyl-1H-indazol-6-yl]-meth-(E)-yliden]-3-methylbutan-1-ol (A7), und nachfolgender katalytischer, asymmetrischer Hydrierung erhalten werden.

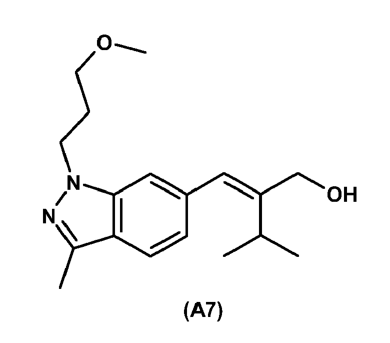

### Beispiel A7:

### 2-[1-[1-(3-Methoxy-propyl)-3-methyl-1H-indazol-6-yl]-meth-(E)-yliden]-3-methyl-butan-1-ol

### b) Herstellung ausgehend von 2-[1-[1-(3-Methoxy-propyl)-3-methyl-1H-indazol-6-yl]-meth-(E)-yliden]-3-methyl-buttersäure ethylester (A3)

Eine Lösung von 19.68 g 2-[1-[1-(3-Methoxy-propyl)-3-methyl-1 H-indazol-6-yl]-meth-(E)-yliden]-3-methyl-buttersäure ethylester (A3) in 433 ml Toluol wird auf -20°C gekühlt und mit einer Lösung von 115.2 ml Diisobutylaluminiumhydrid (1.7 M in Toluol) versetzt, wobei die Temperatur bei -20°C gehalten wird. Das Reaktionsgemisch wird anschliessend auf Raumtemperatur erwärmt und während 1 Stunden bei Raumtemperatur gerührt. Das Reaktionsgemisch wird anschliessend langsam mit 1 l 1M HCl versetzt, wobei die Temperatur unter 30°C gehalten wird. Die Phasen werden getrennt und die wässrige Phase wird mit Diethylether (1 X 1l, 2 X 300 ml) extrahiert. Die vereinigten organischen Phasen werden nacheinander je mit 1 l Wasser, gesättigter wässriger Natriumcarbonat-Lösung und Sole gewaschen, mit Natriumsulfat getrocknet, filtriert und am Rotationsverdampfer eingedampft und am Hochvakuum getrocknet. Aus dem Rückstand wird mittels Flashchromatographie (SiO₂ 60F, Dichlormethan-Methanol-Ammoniak conz 200:5:1) die reine Titelverbindung A7 als gelbes Oel erhalten (14.24 g, 82%). Rf = 0.29 (Dichlormethan-Methanol-Ammoniak conz 200:5:1); Rt = 3.96 (Gradient I).

Nach dem im Beispiel A beschriebenen Verfahren können analog die folgenden Verbindungen hergestellt werden:

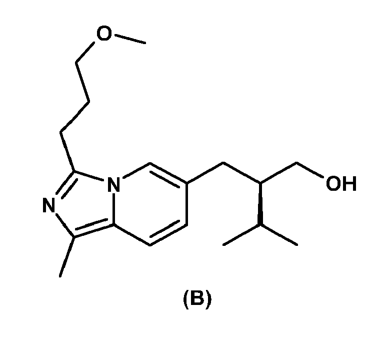

### B) (R)-2-[3-(3-Methoxy-propyl)-1-methyl-imidazo[1,5-a]pyridin-6-ylmethyl]-3-methyl-butan-1-ol

Mischung aus leicht gelblichem Oel und Kristallen; Rf = 0.15 (Essigester-Methanol 95:5); Rt = 3.29 (Gradient IV).

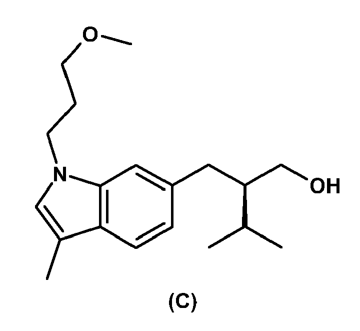

### C) (R)-2-[1-(3-Methoxy-propyl)-3-methyl-1H-indol-6-ylmethyl]-3-methyl-butan-1-ol

Gelbliches Oel Rf = 0.29 (Essigester-Heptan 1:1); Rt = 4.96 (Gradient I).

### Beispiel L:

### Herstellung von (R_{C},S_{Fc},S_{P})-1-[2-(1-dimethylaminoethyl)ferrocen-1-yl]phenylphosphino-1'-bromoferrocene der Formel (A1) [Ph = Phenyl; Me = Methyl]

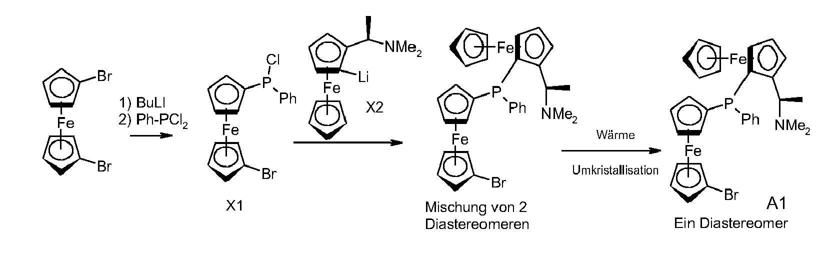

### a) Herstellung 1-Phenyl-chlorphosphin-1'-brom-ferrocen (X1)

Zu einer Lösung von 8g (23,2 mmol) 1,1'-Dibromferrocen in 30 ml Tetrahydrofuran (THF) werden bei einer Temperatur von < -30 °C 14,5 ml (23,2 mmol) (n-Butyllithium (n-Bu-Li) (1,6 M in Hexan) getropft. Es wird 30 Minuten bei dieser Temperatur weitergerührt Dann wird auf -78 °C gekühlt und es werden 3,15 ml (23,2 mmol) Phenyl-dichlorphosphin so langsam zugetropft, dass die Temperatur nicht über -60 °C ansteigt. Nach weiteren 10 Minuten Rühren bei -78 °C lässt man die Temperatur auf Raumtemperatur ansteigen und rührt noch eine Stunde nach. Man erhält so eine Suspension des Monochlorphosphins X1.

### b) Herstellung von L (Gemisch von Diastereomeren)

Zu einer Lösung von 5,98 g (23,2 mmol) (R)-1-Dimethylamin-1-ferrocenyl-ethan in 40 ml Diethylether (DE) werden bei <-10 °C 15,5 ml (23,2 mmol) t-Butyllithium (t-Bu-Li) (1,5 M in Pentan) getropft. Nach 10 Minuten Rühren bei der gleichen Temperatur lässt man die Temperatur auf Raumtemperatur ansteigen und rührt noch 1,5 Stunden nach. Man erhält so eine Lösung der Verbindung X2, die so langsam über eine Kanüle zur gekühlten Suspension des Monochlorphosphins X1 gegeben wird, dass die Temperatur -30 °C nicht übersteigt. Nach weiteren 10 Minuten Rühren bei -30 °C lässt man die Temperatur auf 0°C ansteigen und rührt bei dieser Temperatur noch 2 Stunden nach. Das Reaktionsgemisch wird mit 20 ml Wasser versetzt. Die organische Phase wird abgetrennt, mit Natriumsulfat getrocknet, und das Lösungsmittel unter reduziertem Druck im Rotationsverdampfer abdestilliert. Nach chromatographischer Reinigung (Silikagel 60; Laufmittel = Heptan/Ethylacetat(EA)/NEthyl₃(Net₃) 85:10:5) werden 11,39 g des gewünschten Produkts als Gemisch von 2 Diastereomeren erhalten.

### c) Herstellung von L (ein Diastereomer)

Das gemäss Verfahrensstufe b) erhaltene Produkt wird in 50 ml Toluol gelöst und während 4 Stunden am Rückfluss erhitzt. Nach Abdestillieren des Toluols wird der Rückstand in Ethanol umkristallisiert. Man erhält die Verbindung A1 als gelbe Kristalle und als reines Diastereomer mit einer Ausbeute von 59% der Theorie. ³¹P-NMR (121,5 MHz), CDCl₃): δ-35,3 (s).

### Beispiel L1:

### Herstellung von (R_{C},S_{Fc},S_{P})-1-[2-(1-dimethylaminoethyl)ferrocen-1-yl]phenylphosphino-1'-dicyclohexylphosphinoferrocen (L1) der Formel

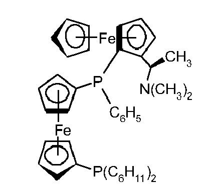

Zu einer Lösung von 629 mg (1 mmol) der Verbindung L in 5 ml Tertiärbutyl-methylether (TBME) werden bei einer Temperatur unter 0 °C 0,85 ml (1,1 mmol) Sekundärbutyllithium (s-Bu-Li) (1,3 M in Cyclohexan) getropft. Man lässt die Temperatur bei 0 °C, rührt 1 Stunde weiter und gibt dann 0,24 ml (1,1 mmol) Chlordicyclohexylphosphin zu. Man lässt die Temperatur auf Raumtemperatur ansteigen, rührt 2 Stunden weiter und versetzt dann das Reaktionsgemisch mit 5 ml einer gesättigten wässerigen NaHCO₃-Lösung. Die organische Phase wird abgetrennt, mit Natriumsulfat getrocknet, und das Lösungsmittel unter reduziertem Druck am Rotationsverdampfer abdestilliert. Nach chromatographischer Reinigung (Silikagel 60; Laufmittel = Heptan/EA/NEt₃ 85:10:5) und Umkristallisation in Methanol wird die Verbindung L1 als orange Kristalle und als reines Diastereomer in einer Ausbeute von 95% der Theorie erhalten. ³¹P NMR (121,5 MHz, CDCl₃): δ -35,4 (s), -6,5 (s).

### Beispiel M1: Herstellung eines Rhodiumkomplexes (NBD ist Norbornadien)

11 mg ( 0,0148 mmol) Ligand L1 und 5,4 mg (0,0144 mmol) [Rh(NBD)₂]BF₄ werden in 0,8 ml CD₃OD gelöst und während 10 Minuten gerührt. Die Lösung wird zur Messung in ein NMR-Rohr transferiert. ³¹P NMR (121.5 MHz, CD₃OD): Zwei übereinander liegende Dublette. Mögliche Zuordnung: δ 26,60 (d, J_{Rh-P} = 163 Hz), 26,30 (d, J_{Rh-P} = 157 Hz).

Die Herstellung der übrigen Metallkomplexe erfolgt in analoger Weise.

## Patentansprüche

1. Verfahren zur Herstellung einer Verbindung der Formel I 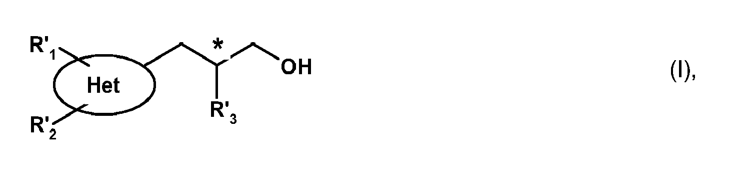 worin Het ein bicyclisches, über ein C-Atom an das Restmolekül geknüpftes, ungesättigtes Heterocyclyl bedeutet, wobei der nicht direkt an das Restmolekül gebundene Ring durch R'₁ und R'₂ substituiert ist, R'₁ und R'₂ unabhängig voneinander H, C₁-C₈-Alkyl, Halogen, Polyhalogen-C₁-C₈-alkoxy, Polyhalogen-C₁-C₈-alkyl, C₁-C₈-Alkoxy, C₁-C₈-Alkoxy-C₁-C₈-alkyl, oder C₁-C₈-Alkoxy-C₁-C₈-alkoxy darstellen, wobei R'₁ und R'₂ nicht gleichzeitig H bedeuten, R'₃ C₁-C₈-Alkyl bedeutet und worin das Kohlenstoffatom, an welches der Rest R'₃ gebunden ist, entweder (R)- oder (S)-Konfiguration aufweist, wobei die (R)-Konfiguration bevorzugt ist, das **dadurch gekennzeichnet ist, dass** man
a) eine Verbindung der Formel II 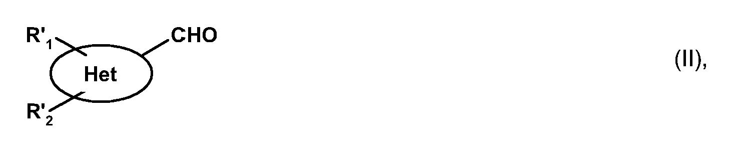 worin Het, R'₁ und R'₂ die zuvor angegebenen Bedeutungen haben, mit einer Verbindung der Formel III, 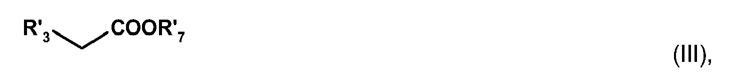 worin R'₃ die zuvor angegebene Bedeutung hat, zu einem Diastereomeren-Gemisch der Formel IV umsetzt, 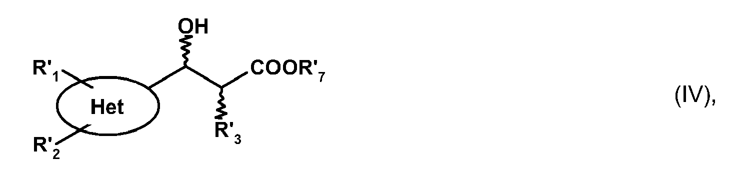 worin R'₇ für C₁-C₁₂-Alkyl, C₃-C₈-Cycloalkyl, Phenyl oder Benzyl steht,
b) die OH-Gruppe des Diastereomeren-Gemisches der Formel IV in eine Abgangsgruppe überführt und die eine Abgangsgruppe enthaltende Verbindung in Gegenwart einer starken Base zu einem Acrylsäureester der Formel V eliminiert, 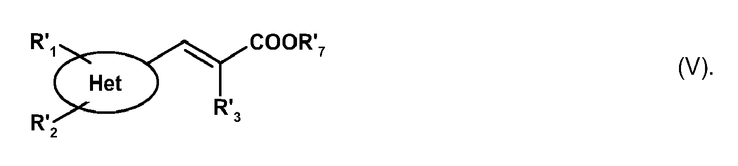 anschliessend entweder gemäss Verfahrensvariante 1
1c) den Acrylsäureester der Formel V durch Verseifung zu einer Verbindung der Formel VI umsetzt, 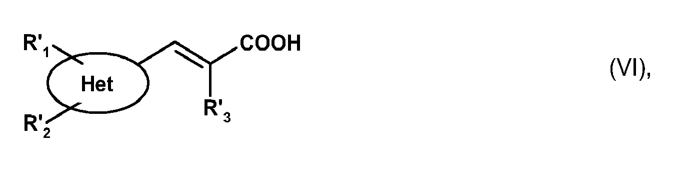
1d) die Säure der Formel VI in Gegenwart von Wasserstoff und katalytischen Mengen eines Metallkomplexes als asymmetrischer Hydrierkatalysator, der Metalle aus der Gruppe Ruthenium, Rhodium oder Iridium enthält, an die chirale, zweizähnige Liganden vom Typ der Ferrocen-1,1'-diphosphine, die in 1-Stellung eine Ferrocen-substituierte, sekundäre Phosphingruppe und in 1'-Stellung eine sekundäre Phosphingruppe aufweisen, gebunden sind, zu einer Verbindung der Formel VII hydriert, 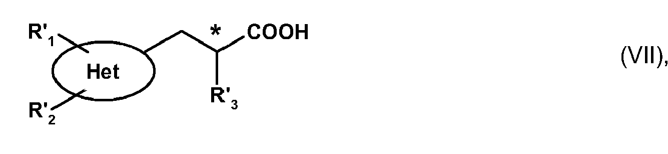 und
1e) die Säure der Formel VII zu einer Verbindung der Formel I reduziert;
oder gemäss Verfahrensvariante 2
2c) den Acrylsäureester der Formel V durch Verseifung zu einer Verbindung der Formel VI umsetzt, 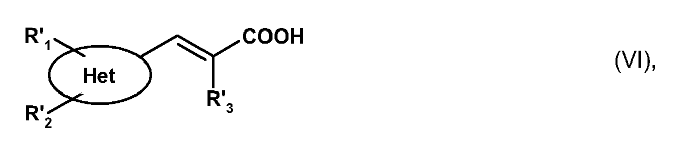
2d) die Säure der Formel VI, zu einer Verbindung der Formel VIII reduziert 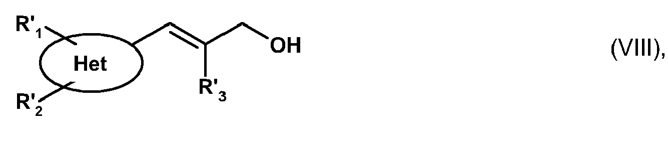 und
2e) den Alkohol der Formel VIII in Gegenwart von Wasserstoff und katalytischen Mengen eines Metallkomplexes als asymmetrischer Hydrierkatalysator, der Metalle aus der Gruppe Ruthenium, Rhodium oder Iridium enthält, an die chirale, zweizähnige Liganden vom Typ der Ferrocen-1,1'-diphosphine, die in 1-Stellung eine Ferrocen-substituierte, sekundäre Phosphingruppe und in 1'-Stellung eine sekundäre Phosphingruppe aufweisen, gebunden sind, zu einer Verbindung der Formel I hydriert;
oder gemäss Verfahrensvariante 3
3c) den Acrylsäureester der Formel V zu einer Verbindung der Formel VIII reduziert 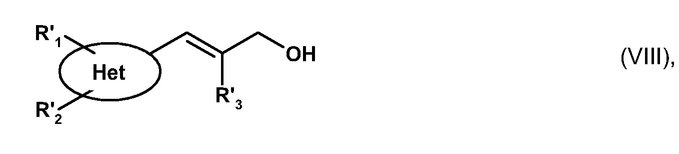 und
3d) den Alkohol der Formel VIII in Gegenwart von Wasserstoff und katalytischen Mengen eines Metallkomplexes als asymmetrischer Hydrierkatalysator, der Metalle aus der Gruppe Ruthenium, Rhodium oder Iridium enthält, an die chirale zweizähnige Liganden vom Typ der Ferrocen-1,1'-diphosphine, die in 1-Stellung eine Ferrocen-substituierte, sekundäre Phosphingruppe und in 1'-Stellung eine sekundäre Phosphingruppe aufweisen, gebunden sind, zu einer Verbindung der Formel I hydriert.

2. Verfahren gemäss Anspruch 1 zur Herstellung einer Verbindung der Formel I, wobei R'₁ Methoxy- oder Ethoxy-C₁-C₄-Alkyl, und R'₂ Methyl, Ethyl, Methoxy oder Ethoxy darstellt.

3. Verfahren gemäss einem der Ansprüche 1 oder 2 zur Herstellung einer Verbindung der Formel I, wobei Het einen bicyclischen, ungesättigten heterocyclischen Rest mit 1 bis 4 Stickstoff- und/oder 1 oder 2 Schwefel- oder Sauerstoffatomen, bestehend aus jeweils 5-und/oder 6-gliedrigen Ringen, darstellt.

4. Verfahren gemäss einem der Ansprüche 1 bis 3 zur Herstellung einer Verbindung der Formel I, wobei mit R'₁ und R'₂ substituiertes Het 1-(3-Methoxy-propyl)-3-methyl-1 H-indol-6-yl, 3-(3-Methoxy-propyl)-1-methyl-imidazo[1,5-a]pyridin-6-yl oder 1-(3-Methoxy-propyl)-3-methyl-1H-indazol-6-yl und R'₃ Isopropyl darstellt.

5. Verfahren gemäss einem der Ansprüche 1 bis 3 zur Herstellung einer Verbindung der Formel I, wobei als Metallkomplexe im Schritt 1d, Metallkomplexe der Formeln IX oder IXa verwendet werden,
[LM¹X¹X²] (IX),
[LM¹X¹]⁺E⁻ (IXa),
worin
M¹ Rhodium oder Iridium bedeutet;
X¹ für zwei Olefine oder ein Dien steht;
X² Cl, Br oder I bedeutet;
E⁻ das Anion einer Sauerstoffsäure oder Komplexsäure darstellt; und
L für einen chiralen Liganden aus der Gruppe der Ferrocen-1,1'-diphosphine, die in 1-Stellung eine Ferrocen-substituierte, sekundäre Phosphingruppe und in 1'-Stellung eine sekundäre Phosphingruppe aufweisen, steht.

6. Verfahren gemäss Anspruch 5 zur Herstellung einer Verbindung der Formel I, wobei als Ligand L eine Verbindung der Formel X in Form von enantiomerenreinen Diastereomeren oder einem Gemisch von Diastereomeren verwendet wird, 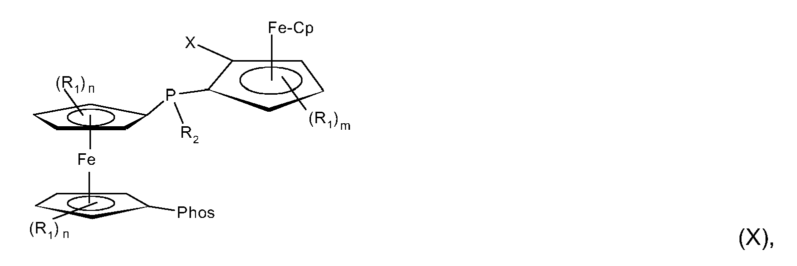 worin
die R₁ gleich oder verschieden sind und C₁-C₄-Alkyl bedeuten;
m für 0 steht oder eine ganze Zahl von 1 bis 3 darstellt;
n für 0 steht oder eine ganze Zahl von 1 bis 4 darstellt;
R₂ einen Kohlenwasserstoffrest oder C-gebundenen Heterokohlenwasserstoffrest bedeutet; Cp für unsubstituiertes oder mit C₁-C₄-Alkyl substituiertes Cyclopentadienyl steht;
X eine C-gebundene, Metalle von Metallierungsreagenzien in die Orthostellung dirigierende, chirale Gruppe darstellt; und
Phos einen P-gebundenen P(III)-Substituenten bedeutet.

7. Verfahren gemäss einem der Ansprüche 1 bis 3 zur Herstellung einer Verbindung der Formel I, wobei als Metallkomplexe im Schritt 2e bzw. 3d, Metallkomplexe der Formeln XI oder XIa verwendet werden,
[LM²X¹X²] (XI),
[LM²X¹]⁺E⁻ (XIa),
worin
M² Rhodium bedeutet;
X¹ für zwei Olefine oder ein Dien steht;
X² Cl, Br oder I bedeutet;
E⁻ das Anion einer Sauerstoffsäure oder Komplexsäure darstellt; und
L für einen chiralen Liganden aus der Gruppe der Ferrocen-1,1'-diphosphine, die in 1-Stellung eine Ferrocen-substituierte, sekundäre Phosphingruppe und in 1'-Stellung eine sekundäre Phosphingruppe aufweisen, steht.

8. Verfahren gemäss Anspruch 7 zur Herstellung einer Verbindung der Formel I, wobei als Ligand L eine Verbindung der Formel X in Form von enantiomerenreinen Diastereomeren oder einem Gemisch von Diastereomeren verwendet wird, 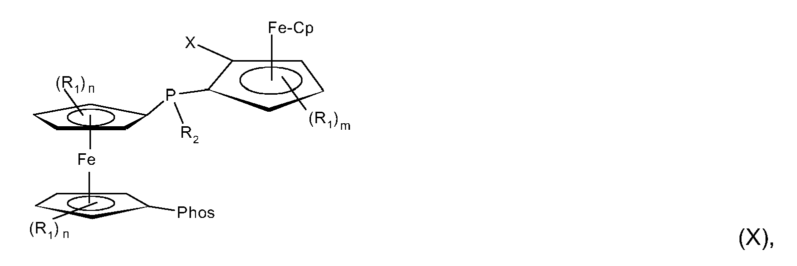 worin
die R₁ gleich oder verschieden sind und C₁-C₄-Alkyl bedeuten;
m für 0 steht oder eine ganze Zahl von 1 bis 3 darstellt;
n für 0 steht oder eine ganze Zahl von 1 bis 4 darstellt;
R₂ einen Kohlenwasserstoffrest oder C-gebundenen Heterokohlenwasserstoffrest bedeutet; Cp für unsubstituiertes oder mit C₁-C₄-Alkyl substituiertes Cyclopentadienyl steht;
X eine C-gebundene, Metalle von Metallierungsreagenzien in die Orthostellung dirigierende, chirale Gruppe darstellt; und
Phos einen P-gebundenen P(III)-Substituenten bedeutet.

9. Verfahren gemäss Anspruch 6 oder 8 zur Herstellung einer Verbindung der Formel I, wobei als Ligand L, eine Verbindung der Formel 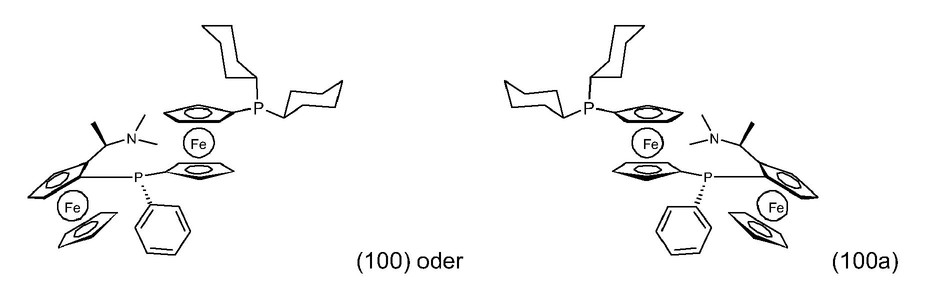 verwendet wird.

10. Verfahren zur Herstellung einer Verbindung der Formel A 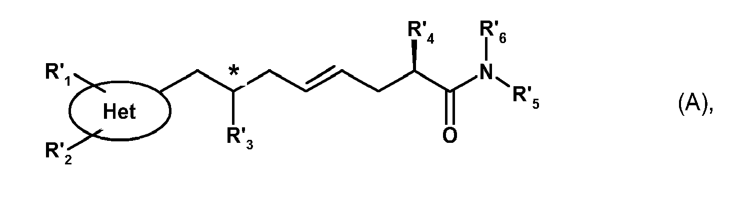 worin Het ein bicyclisches, über ein C-Atom an das Restmolekül geknüpftes, ungesättigtes Heterocyclyl bedeutet, wobei der nicht direkt an das Restmolekül gebundene Ring durch R₁ und R₂ substituiert ist, R₁ und R₂ unabhängig voneinander H, C₁-C₈-Alkyl, Halogen, Polyhalogen-C₁-C₈-alkoxy, Polyhalogen-C₁-C₈-alkyl, C₁-C₈-Alkoxy, C₁-C₈-Alkoxy-C₁-C₈-alkyl, oder C₁-C₈-Alkoxy-C₁-C₈-alkoxy darstellen, wobei R₁ und R₂ nicht gleichzeitig H bedeuten, R₃ C₁-C₈-Alkyl bedeutet, R₄ für C₁-C₈-Alkyl steht, R₅ C₁-C₈-Alkyl oder C₁-C₈-Alkoxy bedeutet, R₆ C₁-C₈-Alkyl darstellt, oder R₅ und R₆ zusammen gegebenenfalls mit C₁-C₄-Alkyl, Phenyl oder Benzyl substituiertes Tetramethylen, Pentamethylen, 3-Oxa-1,5-pentylen oder -CH₂CH₂O-C(O)- sind und worin das Kohlenstoffatom, an welches der Rest R'₃ gebunden ist, entweder (R)- oder (S)-Konfiguration aufweist, wobei die (R)-Konfiguration bevorzugt ist, **dadurch gekennzeichnet, dass** man eine Verbindung der Formel B 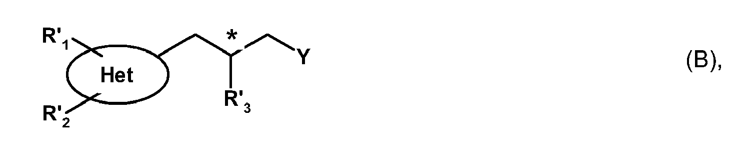 mit einer Verbindung der Formel C, 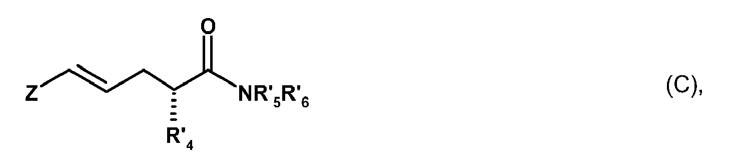 worin Het und R'₁ bis R'₆ die zuvor angegebenen Bedeutungen haben, Y Cl, Br oder I und Z Cl, Br oder I bedeuten und worin das Kohlenstoffatom, an welches der Rest R'₃ gebunden ist, entweder (R)- oder (S)-Konfiguration aufweist, wobei die (R)-Konfiguration bevorzugt ist, in Gegenwart eines Alkali- oder Erdalkalimetalls umsetzt,
wobei die Verbindung der Formel B durch Halogenierung einer gemäss Anspruch 1 hergestellten Verbindung der Formel I hergestellt wird.
